# FASCICULE DE BREVET EUROPEEN

(11) **EP 1 556 382 B1**
(45) Date de publication et mention de la délivrance du brevet: **28.05.2008**
(21) Numéro de dépôt: 03810495.6
(22) Date de dépôt: 21.10.2003
(51) Int. Cl.: C07D 471/04, A61K 31/437, A61P 35/00, C07D 471/16

(54) **DERIVES DE PYRIDOINDOLONE SUBSTITUES EN -3 PAR UN PHENYLE, LEUR PREPARATION ET LEUR APPLICATION EN THERAPEUTIQUE.**
3-PHENYLPYRIDOINDOLDERIVATE, IHRE HERSTELLUNG UND VERWENDUNG
3-PHENYL SUBSTITUTED PYRIDOINDOLONE, PREPARATION AND THERAPEUTIC USE THEREOF

(30) Priorité: 23.10.2002 FR 0213264
(43) Date de publication de la demande: 27.07.2005
(73) Titulaire: Sanofi-Aventis, 75013 Paris (FR)
(72) Inventeur: BOURRIE, Bernard, F-34980 Saint-Gély-du-Fesc (FR); CASELLAS, Pierre, F-34070 Montpellier (FR); CIAPETTI, Paola, F-67120 Altorf (FR); DEROCQ, Jean-Marie, F-34570 Murviel-les-Montpellier (FR); JEGHAM, Samir, FR-34980 Montferrier-Sur-Lez (FR); MUNEAUX, Yvette, F-34270 Les Matelles (FR); WERMUTH, Camille-Georges, F-67100 Strasbourg (FR)
(74) Mandataire: Le Pennec, Magali
(86) Numéro de dépôt international: PCT/FR2003/003110
(87) Numéro de publication internationale: WO 2004/041817

(56) Documents cités:
- WO-A-01/09129
- WO-A-02/087574

## Description

La présente invention se rapporte à des dérivés de pyridoindolone substitués en -3 par un phényle, à leur préparation et à leur application en thérapeutique.

### L'art antérieur

Le brevet français 276 5581 décrit des composés de formule : 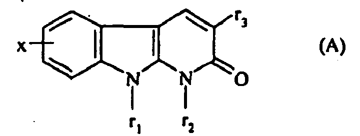 dans laquelle : x représente un atome d'hydrogène ou de chlore ou un groupe méthyle ou méthoxy ; r₁ représente un atome d'hydrogène ou un groupe méthyle ou éthyle ; r₂ représente un groupe méthyle ou éthyle ; ou bien r₁ et r₂ forment ensemble un groupe (CH₂)₃ ; r₃ représente, soit un groupe phényle éventuellement substitué par un atome d'halogène ou un groupe méthyle ou méthoxy, soit un groupe thiényle. Dans la description de ce brevet, il est mentionné que les composés de formule (A) ayant une affinité pour les sites modulateurs oméga associés aux récepteurs GABA_{A}, peuvent être utilisés dans le traitement d'affections liés aux désordres de la transmission gabaergique associés aux sous-types de récepteurs GABA_{A}, tels que l'anxiété, les troubles du sommeil, l'épilepsie etc...

La demande internationale WO-A-01 09129 décrit des composés pour lesquels le squelette de base est différent de celui des pyridoindolones revendiqués.

La demande internationale WO-A-02 087574 décrit des pyridoindolones qui diffèrent des composés de la présence invention par le substituant r3. r3 peut être un groupe phényle R3 éventuellement monosubstitué ou bien un groupe thiényle (formules (I) et (Ibis)). Dans le cas où le groupe phényle est substitué, l'unique substituant peut être un atome d'halogène, un groupe méthyle ou un groupe méthoxy.

La présente invention a pour objet des composés ayant une activité anticancéreuse, répondant à la formule : 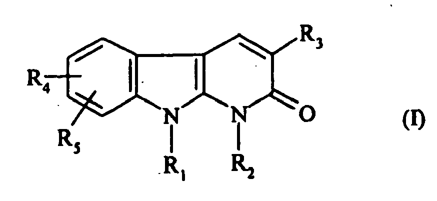 dans laquelle :
- R₁ représente un atome d'hydrogène, un groupe (C₁-C₄)alkyle ou un groupement (CH₂)ₙOH, (CH₂)ₙ-O-tétrahydropyran-2-yle, (CH₂)ₙNR'₆R'₇, (CH₂)ₙCN, (CH₂)ₙCO₂(C₁-C₄)Alk ou (CH₂)ₙCONR₆R₇;
- R₂ représente un atome d'hydrogène ou un groupe (C₁-C₄)alkyle ;
- ou R₁ et R₂ ensemble forment un groupe (CH₂)₃ ;
- R₃ représente un phényle monosubstitué par un groupe hydroxyle, hydroxyméthyle, carboxy, (C₁-C₄)alcanoyle, azido, (C₁-C₄)alcoxycarbonyle, hydroxyiminométhyle, (C₁-C₄)alkylsulfonyle, trifluorométhyle, thiole, (C₁-C₄)alkylthio, cyano ou par un groupement (CH₂)ₘNR'₇R₁₀, CONR₆R₈ ou O(CH₂)ₙR₉ ; un phényle substitué par 2 à 5 substituants identiques ou différents choisis parmi un atome d'halogène, un groupe (C₁-C₄)alkyle, trifluorométhyle, hydroxyle, hydroxyméthyle, (C₁-C₄)alcoxy, carboxy, (C₁-C₄)alcanoyle, azido, (C₁-C₄)alcoxycarbonyle, hydroxyiminométhyle, thiole, (C₁-C₄)alkylthio, (C₁-C₄)alkylsulfonyle, un phényle, cyano ou par un groupement (CH₂)ₘNR'₇R₁₀, CONR₆R₈ ou O(CH₂)ₙR₉ ; ou R₃ représente un groupe benzodioxolyle non substitué ou substitué sur le phényle par un atome d'halogène ;
- R₄ et R₅ sont identiques ou différents et représentent chacun indépendamment un atome d'hydrogène ou d'halogène ou un groupe hydroxyle, (C₁-C₄)alkyle, trifluorométhyle, phényle, cyano, (C₁-C₄)alcoxy, (C₁-C₄)alcoxycarbonyle, (C₁-C₄)alkylsulfonyle ou un groupement O-(CH₂)ₙNR₆R₇ ou (CH₂)ₙNR₆R₇ ;
- R₆ représente l'hydrogène ou un groupe (C₁-C₄)alkyle ;
- R₇ représente l'hydrogène ou un groupe (C₁-C₄)alkyle ;
- ou R₆ et R₇ ensemble avec l'atome d'azote auquel ils sont liés constituent un radical hétérocyclique choisi parmi : pipéridyle, morpholinyle, pyrrolidinyle, pipérazinyle ou 4-méthylpipérazin-1-yle ;
- R'₆ représente l'hydrogène ou un groupe (C₁-C₄)alkyle ;
- R'₇ représente l'hydrogène ou un groupe (C₁-C₄)alkyle ;
- ou R'₆ et R'₇ ensemble avec l'atome d'azote auquel ils sont liés constituent un radical hétérocyclique choisi parmi morpholinyle ou pyrrolidinyle ;
- R₈ représente l'hydrogène, un groupe (C₁-C₄)alkyle ou un groupement -(CH₂)ₙNR₆R₇ ;
- ou R₆ et R₈ ensemble avec l'atome d'azote auquel ils sont liés constituent un radical hétérocyclique choisi parmi : pipéridyle, morpholinyle, pyrrolidinyle, pipérazinyle ou 4-méthylpipérazin-1-yle ;
- R₉ représente un radical phényle ou un groupe amino, morpholin-4-yle, cyano, ou (C₁-C₄)alcoxycarbonyle ;
- R₁₀ représente R'₆ ou un groupe phényle, pyridyle ou pyrimidinyle ou un groupement (CH₂)ₙNR'₆R'₇ ;
- ou R'₇ et R₁₀ ensemble avec l'atome d'azote auquel ils sont liés constituent un radical hétérocyclique choisi parmi pipérazinyle ou 4-méthylpipérazin-2-yle ;
- n représente 1, 2 ou 3 ;
- m représente 0 ou 1.
- Alk représente un alkyle.

Les composés de formule (I) peuvent exister à l'état de bases ou de sels d'addition à des acides. De tels sels d'addition font partie de l'invention.
Ces sels sont avantageusement préparés avec des acides pharmaceutiquement acceptables, mais les sels d'autres acides utiles, par exemple, pour la purification ou l'isolement des composés de formule (I) font également partie de l'invention.

Les composés de formule (I) peuvent également exister sous forme d'hydrates ou de solvats, à savoir sous forme d'associations ou de combinaisons avec une ou plusieurs molécules d'eau ou avec un solvant. De tels hydrates et solvats font également partie de l'invention.

Dans le cadre de la présente invention, on entend par :
- un atome d'halogène : un fluor, un chlore, un brome, ou un iode ;
- un groupe (C₁-C₄)alkyle : un groupe aliphatique saturé linéaire ou ramifié comportant 1 à 4 atomes de carbone. A titre d'exemple, on peut citer les groupes méthyle, éthyle, propyle, isobutyle, butyle, isobutyle, *tert*-butyle ;
- un groupe (C₁-C₄)alcoxy : un radical O-alkyle où le groupe alkyle est tel que précédemment défini.

La présente invention a tout particulièrement pour objet des composés de formule (I) dans laquelle :
- R₁ représente un atome d'hydrogène, un groupe (C₁-C₄)alkyle ou un groupement (CH₂)ₙCO₂(C₁-C₄)Alk ou (CH₂)ₙCONR₆R₇ ;
- R₂ représente un atome d'hydrogène ou un groupe (C₁-C₄)alkyle ;
- R₃ représente un phényle monosubstitué par un groupe hydroxyle, hydroxyméthyle, carboxy, (C₁-C₄)alcoxycarbonyle, hydroxyiminométhyle, (C₁-C₄)alkylsulfonyle, trifluorométhyle, thiole, (C₁-C₄)alkylthio, cyano ou par un groupement (CH₂)ₘNR₆R₇ ou CONR₆R₈ ; un phényle substitué par 2 à 5 substituants identiques ou différents choisis parmi un atome d'halogène, un groupe (C₁-C₄)alkyle, trifluorométhyle, hydroxyle, hydroxyméthyle, (C₁-C₄)alcoxy, carboxy, (C₁-C₄)alcoxycarbonyle, hydroxyiminométhyle, thiole, (C₁-C₄)alkylthio, (C₁-C₄)alkylsulfonyle, cyano ou par un groupement (CH₂)ₘNR₆R₇ ou CONR₆R₈ ; ou R₃ représente un groupe benzodioxolyle ;
- R₄ et R₅ sont identiques ou différents et représentent chacun indépendamment un atome d'hydrogène ou d'halogène ou un groupe hydroxyle, (C₁-C₄)alkyle, trifluorométhyle, cyano, (C₁-C₄)alcoxy, (C₁-C₄)alcoxycarbonyle ou un groupement O-(CH₂)ₙNR₆R₇ ;
- R₆ représente l'hydrogène ou un groupe (C₁-C₄)alkyle ;
- R₇ représente l'hydrogène ou un groupe (C₁-C₄)alkyle ;
- ou R₆ et R₇ ensemble avec l'atome d'azote auquel ils sont liés constituent un radical hétérocyclique choisi parmi : pipéridyle, morpholinyle, pyrrolidinyle, pipérazinyle ou 4-méthylpipérazin-1-yle;
- R₈ représente l'hydrogène, un groupe (C₁-C₄)alkyle ou un groupement -(CH₂)ₙNR₆R₇;
- ou R₆ et R₈ ensemble avec l'atome d'azote auquel ils sont liés constituent un radical hétérocyclique choisi parmi : pipéridyle, morpholinyle, pyrrolidinyle, pipérazinyle ou 4-méthylpipérazin-1-yle ;
- n représente 1, 2 ou 3 ;
- m représente 0 ou 1.
- Alk représente un alkyle.

Parmi les composés de formule (I) objets de l'invention, on peut citer les composés préférés qui se définissent comme suit :
- R₁ représente un atome d'hydrogène, un groupe méthyle, cyanométhyle, (C₁-C₄)alcoxycarbonylméthyle, aminométhyle, aminoéthyle, aminopropyle, pyrrolidinoéthyle ;
- et/ou R₂ représente un groupe méthyle ;
- et/ou R₁ et R₂ ensemble forment un groupe (CH₂)₃ ;
- et/ou R₃ représente un phényle monosubstitué par un groupe hydroxyle, (C₁-C₄)alcoxycarbonyle, méthylsulfonyle, trifluorométhyle, méthylthio, cyanométhoxy, aminoéthoxy, acétyle, hydroxyméthyle, cyano, amino, azido, aminométhyle, hydroxyiminométhyle ou un groupe (CH₂)ₘNR'₇R₁₀ dans lequel R'₇ représente un atome d'hydrogène ou un méthyle, R₁₀ représente un atome d'hydrogène ou un groupe phényle, pyridyle, pyrimidinyle ou R'₇ et R₁₀ ensemble avec l'atome d'azote auquel ils sont liés constituent un groupe pipérazin-1-yle ou 4-méthylpipérazin-1-yle et m représente zéro ou un ; ou R₃ représente un phényle substitué par 2 à 3 substituants identiques ou différents choisis parmi un atome d'halogène, un groupe méthyle, méthoxy, méthylthio, trifluorométhyle, hydroxyle, (C₁-C₄)alcoxycarbonyle, méthylsulfonyle, cyanométhoxy, aminoéthoxy, acétyle, hydroxyméthyle, cyano, amino, azido, aminométhyle, hydroxyiminométhyle ou un groupe (CH₂)ₘNR'₇R₁₀ dans lequel R'₇ représente un atome d'hydrogène ou un méthyle, R₁₀ représente un atome d'hydrogène ou un groupe phényle, pyridyle, pyrimidinyle ou R'₇ et R₁₀ ensemble avec l'atome d'azote auquel ils sont liés constituent un groupe pipérazin-1-yle ou 4-méthylpipérazin-1-yle et m représente zéro ou un ; ou R₃ représente un groupe benzodioxolyle non substitué ou substitué sur le phényle par un atome d'halogène ;
- et/ou R₄ représente un atome d'halogène, un groupe méthyle, méthoxy ou (C₁-C₄)alcoxycarbonyle ;
- et/ou R₅ représente un atome d'hydrogène ou un groupe méthyle.

Tout particulièrement, on préfère les composés suivants :
- Acide 3-(2,4-diméthoxyphényl)-1,9-diméthyl-2-oxo-2,9-dihydro-1*H*-pyrido[2,3-b]indole-6-carboxylique ;
- 3-(2,4-diméthoxyphényl)-1,6-diméthyl-1,9-dihydro-2*H*-pyrido[2,3-b] indol-2-one ;
- 3-(3-Hydroxyméthylphényl)-1,6-diméthyl-1,9-dihydro-2*H*-pyrido[2,3-b] indol-2-one.
   .. 3-(2,4-dichlorophényl)-1,6-diméthyl-1,9-dihydro-2*H*-pyrido[2,3-b] indol-2-one ;
- 3-(1,6-diméthyl-2-oxo-2,9-dihydro-1*H*-pyrido[2,3-b]indol-3-yl) benzonitrile ;
- 3-(4-Aminophényl)-1,6-diméthyl-1,9-dihydro-2*H*-pyrido[2,3-b]indol-2-one.
- 3-(6-chloro-1,3-benaodioxol-5-yl)-1,6-diméthyl-1,9-dihydco-2*H*-pyrido[2,3-b]indol-2-one ;
- 1,6-Diméthyl-1,9-dihydro-3-(phénylaminophényl)-2*H*-pyrido[2,3-b] indol-2-one.
- 6-bromo-3-(3,5-diméthylphényl)-1-méthyl-1,9-dihydro-2*H*-pyrido[2,3-b]indol-2-one;
- 1,6-Diméthyl-3-(3-(trifluorométhyl)phényl)-1,9-dihydro-2*H*-pyrido[2,3-b] indol-2-one ;
- 1,6-Diméthyl-3-(3-(pyridin-2-ylamino)phényl)-1,9-dihydro-2*H*-pyrido[2,3-b] indol-2-one ;
- 1,6-Diméthyl-3-(3-pyàmidin-2-ylamino)phényl)-1,9-dihydro-2*H*-pyrido[2,3-b] indol-2-one ;
- 3-(3-Acétylphényl)-1,6-diméthyl-1,9-dihydro-2*H*-pyrido[2,3-b] indol-2-one ;
- 2-(2,4-Dichlorophényl)-9-méthyl-5,6-dihydro-3*H*, 4*H*-3a, 6a-diazafluoroanthen-3-one ;
- 9-(Cyanométhyl)-3-(2,4-dichlorophényl)-2-oxo-2,9-dihydro-1*H*-pyrido[2,3-b]indole-6-carboxylate de méthyle ;
ainsi que leurs sels d'addition, leurs solvats et leurs hydrates.

Dans ce qui suit, on entend par groupe protecteur Gp ou G'p un groupe qui permet, d'une part, de protéger une fonction réactive telle qu'un hydroxy ou une amine pendant une synthèse et, d'autre part, de régénérer la fonction réactive intacte en fin de synthèse. Des exemples de groupes protecteurs ainsi que des méthodes de protection et de déprotection sont données dans « Protective Groups in Organic Synthesis », Green et al., 2nd Edition (John Wiley & Sons, Inc., New York).

On entend par groupe partant, dans ce qui suit, un groupe pouvant être facilement clivé d'une molécule par rupture d'une liaison hétérolytique, avec départ d'une paire électronique. Ce groupe peut ainsi être remplacé facilement par un autre groupe lors d'une réaction de substitution, par exemple. De tels groupes partants sont, par exemple, les halogènes ou un groupe hydroxy activé tel qu'un mésyle, tosyle, triflate, acétyle, etc. Des exemples de groupes partants ainsi que des références pour leur préparation sont donnés dans « Advances in Organic Chemistry », J. March, 3rd Edition, Wiley Interscience, p. 310-316.

Conformément à l'invention, on peut préparer les composés de formule générale (I) selon le procédé qui suit.

Ce procédé est caractérisé en ce que :
on fait réagir un 2-aminoindole de formule : 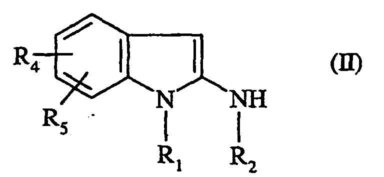
dans laquelle R₁, R₂, R₄ et R₅ sont tels que définis pour un composé de formule (I) avec un ester de formule : 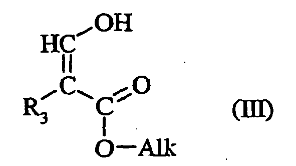 dans laquelle R₃ est tel que défini pour un composé de formule (I) et Alk représente un alkyle en C₁-C₄.

La réaction est effectuée dans un solvant polaire et de préférence basique, par exemple dans la pyridine, à une température comprise entre la température ambiante et la température de reflux du solvant.

D'une manière générale, on peut également préparer selon le procédé de l'invention, un composé de formule : 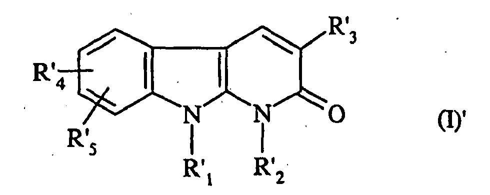 dans laquelle les substituants R'₁, R'₂, R'₃, R'₄, R'₅ sont des précurseurs des substituants R₁, R₂, R₃, R₄, R₅ tels que définis pour un composé de formule (I), puis, en utilisant des méthodes connues de l'homme de l'art, transformer ces substituants pour obtenir les substituants R₁, R₂, R₃, R₄, R₅ désirés pour le composé de formule (I).

A partir d'un composé de formule (I) dans laquelle R₁ ou R₂ est l'hydrogène, on prépare un composé de formule (I) dans laquelle R₁ et/ou R₂ est un groupe alkyle par action d'un iodure d'alkyle en présente de NaH.

Les composés de formule (I) dans laquelle le substituant R₁ est un groupe -(CH₂)ₙCO₂(C₁-C₄)Alk ou -(CH₂)ₙCONR₆R₇ sont préparés à partir des composés correspondants de formule (I)' dans laquelle R₁ = H et R₂, R₃, R₄, R₅ ont les mêmes valeurs.

On peut substituer par exemple, un composé de formule (I) dans laquelle R₁ = H par un groupement (CH₂)₂CN en faisant agir un composé de formule Br(CH₂)ₙCN en présence d'hydrure de sodium.

Par ailleurs, pour préparer un composé de formule (I) dans laquelle R₁ représente un groupe (CH₂)ₙNR'₆R'₇, on peut faire agir un composé bromé de formule Br(CH₂)ₙNR'₆R'₇ sur un composé de formule (I) dans laquelle R₁ = H.

Plus généralement, pour préparer un composé de formule (I) dans laquelle R₁ représente un groupe (CH₂)ₙNR'₆R'₇, on peut faire agir sur un composé de formule (I) dans laquelle R₁ = H, un composé de formule X(CH₂)ₙNGp dans laquelle X représente un groupe partant tel qu'un atome de brome, un groupe mésyle ou tosyle, par exemple et Gp représente un groupe protecteur de l'azote ; après déprotection de l'azote, on peut, le cas échéant, alkyler l'amine formée en utilisant les méthodes connues de l'homme de l'art.

Pour préparer un composé de formule (I) dans laquelle R₁ représente un groupe (CH₂)ₙOH, on peut faire agir un composé de formule X(CH₂)ₙO-G'p dans laquelle X est un groupe partant et G'p est un groupe protecteur de l'oxygène sur un composé de formule (I) dans laquelle R₁ = H, puis traiter le composé ainsi obtenu pour enlever le groupe protecteur par des méthodes connues de l'homme de l'art.

Pour préparer un composé de formule (I) dans laquelle les substituants R₃ et/ou R₄ et/ou R₅ contiennent un groupe hydroxyméthyle, hydroxyiminométhyle, alkylaminométhyle ou dialkylaminométhyle, on procède par transformation du composé de formule (I) correspondant portant un substituant R₃ et/ou R₄ et/ou R₅ contenant un groupe cyano, par des méthodes connues de l'homme de l'art.

Pour préparer un composé de formule (I) dans laquelle les substituants R₃ et/ou R₄ et/ou R₅ contiennent un groupe hydroxyle, on peut préparer d'abord un composé de formule (I) analogue dans lequel les substituants R₃ et/ou R₄ et/ou R₅ contiennent un groupe hydroxyle protégé puis, dans une étape ultérieure, transformer ce groupe en hydroxyle par des méthodes connues de l'homme de l'art. Comme groupe protecteur de l'hydroxyle, on peut utiliser un benzyle, un benzoyle ou un (C₁-C₄)alkyle, par exemple.

Les composés de formule (I) dans laquelle R₄ et/ou R₅ représente un atome de Br ou le(s) substituant(s) sur le groupe phényle R₃ représente un (ou plusieurs) atome(s) de brome peuvent être utilisés comme précurseurs pour préparer d'autres composés selon l'invention par exemple des composés portant des substituants amines tels que (CH₂)ₙNR₆R₇, (CH₂)ₘNR'₇R₁₀ en utilisant des réactions connues de l'homme de l'art.

Les composés portant un substituant bromé sont également utiles pour la préparation des composés portant un substituant alcoxycarbonyle.

Les aminoindoles de formule (II) peuvent être préparés par des méthodes telles que celles décrites dans Khim. Geterosikl. Soedin., 1973, 12, 647-652 et dans J. Heterocycl. Chem., 1975, 12, 135-138.

Certains dérivés de 2-aminoindole de formule (II) sont connus et décrits dans Khim. Geterotsikl. Soedin., 1973, 4, 511-515 ; Eur. J. Med. Chem. Chim. Ther., 1992, 27 (9), 908-918 ; Chem. Heterocycl. Compd. (Engl. Transl.), 1970, 6, 338-343 ; Tetrahedron, 1971, 27, 775-785 ; Pharm. Chem. J. (Engl. Transl.), 1990, 24 (11), 810-812 ; Tetrahedron, Lett., 1996, 37 (28), 4931-4932.

Certains esters de formule (III) sont connus et peuvent être préparés par des méthodes telles que celles décrites dans J. Org. Chem., 1984, 49 (22), 4287-4290 ; J. Am. Chem. Soc., 1974, 96 (7), 2121 ; Tetrahedron, 1970, 26 (2), 715-719 ; Synth. Commun., 2000, 30 (8), 1401-1411 ; Zhongguo Yaowu Huaxue Zazhi, 2000, 10 (1), 9-12, 25 ; JP 19 680 131, EP 260 832, EP 178 826, WO 97-46 577, DE 3 221915.

On peut également préparer les composés selon l'invention par un procédé caractérisé en ce que :
on fait réagir un aminoindole de formule : 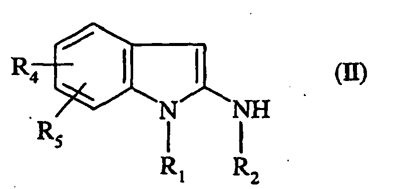
dans laquelle R₁, R₂, R₄ et R₅ sont tels que définis pour un composé de formule (I) avec un ester de formule : 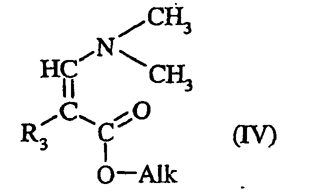 dans laquelle R₃ est tel que défini pour un composé de formule (I) et Alk représente un alkyle en C₁-C₄.

La réaction est effectuée dans un solvant protique et polaire, de préférence en milieu acide, à une température comprise entre la température ambiante et la température de reflux du solvant.

La préparation du composé de formule (IV) est effectuée au moyen du diméthoxy-N,N-diméthylméthanamine (V) d'après une méthode similaire à celle décrite dans J. Org. Chem., 1982, 47, 2846-2851 ou au moyen du réactif de Bredereck (*tert*butoxybis(diméthylamino)méthane) selon J. Org. Chem., 1982, 15, 2846-2851 et selon le schéma réactionnel ci-après :

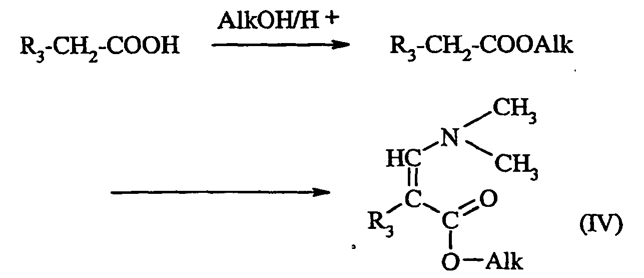

Sauf indication contraire, les spectres de résonance magnétique nucléaire (RMN) du proton sont enregistrés dans DMSO-d₆, la référence est placée dans le DMSO-d₆ qui se situe à 2,50 ppm du tétraméthylsilane.

Les signaux observés en RMN sont exprimés ainsi : s: singulet ; se : singulet élargi ; d : doublet ; d.d : doublet dédoublé ; t : triplet ; td : triplet dédoublé ; q : quadruplet ; m : massif ; mt : multiplet.

Les exemples suivants décrivent la préparation de certains composés conformes à l'invention. Ces exemples ne sont pas limitatifs et ne font qu'illustrer la présente invention. Les numéros des composés exemplifiés renvoient à ceux donnés dans le tableau ci-après, qui illustre les structures chimiques et les propriétés physiques de quelques composés selon l'invention.

Dans les préparations et exemples qui vont suivre, les abréviations suivantes sont utilisées :
TEA : triéthylamine
DMA : diméthylacétamide
DMF : diméthylformamide
DBU: 1,8-diazabicyclo[5.4.0]undec-7-ène
LAH : LiAlH₄ : hydrure de lithium et d'aluminium
NMP : N-Me pyrrolidin-2-one
LiN(TMS)₂ : lithium bis(triméthylsilyl)amide
DCM : dichlorométhane
AcOEt : acétate d'éthyle
AcOH : acide acétique
NBS : N-bromosuccinimide
AIBN : 2,2'-azobis-isobutyrylnitrile
Xantphos : 4,5-bis(diphénylphosphino)-9,9-diméthylxanthène
Pd(dba)₃ : tris(dibenzylidèneacétone)dipalladium
BOP : benzotriazol-1-yloxytris(diméthylamino)phosphonium hexafluorophosphate
MTBE : méthyl *tert*-butyl éther
MiBK : méthyl *iso*-butyl cétone
   réactif de Bredereck : *tert*butoxybis(diméthylamino)méthane
TA : température ambiante

### Préparation des composés de formule (II).

Les composés de formule (II) peuvent exister sous 2 formes tautomères :

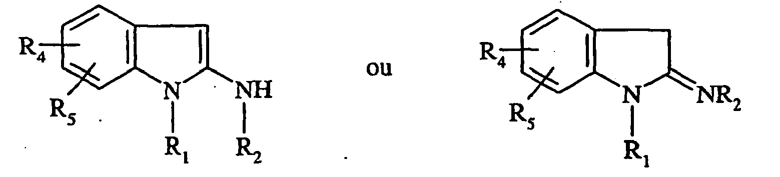

### Préparation 1.1

### Chlorhydrate de N,1,5-triméthyl-1H-indole-2-amine.

### A) N'-(4-méthylphényl)acétohydrazide.

104,8 g de chlorhydrate de 1-(4-méthylphényl)hydrazine sont mis en suspension dans 525 ml d'acétate d'isopropyle, on ajoute une solution de 104,8 g de carbonate de potassium dans 300 ml d'eau puis on agite jusqu'à disparition du solide. Tout en maintenant la température inférieure à 20°C, on ajoute 77,4 g d'anhydride acétique puis on laisse sous agitation à 20°C, on observe la formation d'un précipité qui disparaît lorsque l'on chauffe vers 55-60°C. On lave la phase organique 2 fois par 200 ml d'eau puis on refroidit à 0-5°C pendant une nuit. On récupère le produit formé par filtration puis on le lave 2 fois par 100 ml de MTBE.

RMN CDCl₃ (300 MHz) : 2,02 ppm : s : 3H ; 2,29 ppm : s : 3H ; 6,14 ppm : d : 1H ; 6,73 ppm : d : 2H ; 7,03 ppm : d : 2H ; 7,72 ppm : s : 1H.

### B) N,N'-diméthyl-N'-(4-méthylphényl)acétohydrazine.

On met en suspension 60 g d'hydrazine de l'étape précédente et 11,8 g de bromure de tétrabutylammonium dans 240 ml de toluène et on ajoute 292 g de NaOH à 50 % dans de l'eau puis 155,6 g d'iodure de méthyle. On ajoute alors 83 g de soude en pastilles puis on chauffe le milieu réactionnel à 80°C pendant 6 heures. On refroidit vers 30-35°C puis on ajoute 500 ml d'eau. La phase organique est lavée 3 fois par 100 ml d'eau. La phase organique est séchée par distillation azétropique de l'eau sous pression réduite.

RMN CDCl₃ (300 MHz) : 2,15 ppm : s : 3H ; 2,31 ppm : s : 3H ; 2,95 ppm : s : 3H ; 3,10 ppm : s : 3H ; 6,63 ppm : d : 2H ; 7,13 ppm : d : 2H.

### C) Chlorhydrate de N,1,5-triméthyl-1H-indole-2-amine.

Le produit obtenu à l'étape précédente est dissous dans du toluène et l'on ajoute 61,5 g d'oxychlorure de phosphore et on chauffe à 80°C pendant 2 heures. On ajoute 100 ml d'acétate d'éthyle à 80°C puis on refroidit le milieu à TA. Le précipité est filtré puis lavé 2 fois par 50 ml d'acétate d'éthyle, F = 222°C.

RMN DMSO (200 MHz) : 2,36 ppm : s : 3H ; 3,11 ppm : s : 3H ; 3,49 ppm : s : 3H ; 4,29 ppm : s : 1H ; 7,25-7,35 ppm : m : 3H ; 10,07 ppm : m : 1H.

### Préparation 1.2

### Dichlorhydrate de N,5-diméthyl-1H-indole-2-amine.

### A) N'-(4-méthylphényl)acétohydrazide.

Un autre procédé de préparation de ce composé est décrit ci-après.

On dissout 5 g de chlorhydrate de 1-(4-méthylphényl)hydrazine dans de l'eau puis on ajoute de la triéthylamine jusqu'à neutralisation du sel. On extrait à l'AcOEt puis on évapore à sec. On dissout le précipité formé dans 30 ml d'éther puis on ajoute, goutte à goutte, une solution de 4,6 ml d'anhydride acétique dissous dans 30 ml d'éther. On agite 15 minutes à 0°C puis on filtre le précipité formé pour obtenir 3 g du composé attendu.

RMN CDCl₃ (300 MHz) : 2,02 ppm : s : 3H ; 2,29 ppm : s : 3H ; 6,14 ppm : d : 1H ; 6,73 ppm : d : 2H ; 7,03 ppm : d : 2H ; 7,72 ppm : s : 1H.

### B) N-Méthyl-N'-(4-méthylphényl)acétohydrazide.

On met en suspension 0,8 g de NaH à 60 % dans 30 ml de DMF. On ajoute à 0°C, goutte à goutte, 3,2 g d'hydrazine obtenu à l'étape précédente dans 20 ml de DMF. Lorsqu'il n'y a plus de dégagement gazeux, on ajoute 1,8 ml d'iodure de méthyle et on agite 1 heure à TA. On verse sur une solution de NH₄Cl saturée puis on extrait à l'AcOEt. On lave plusieurs fois avec une solution de NaCl saturée puis on évapore à sec. On purifie par chromatographie sur colonne de silice éluée avec un mélange AcOEt/heptane (25/75 ; v/v) puis (50/50 ; v/v) pour obtenir 1,0 g du composé attendu sous forme de poudre blanche.

RMN CDCl₃ (200 MHz) : 2,21 ppm : s : 3H ; 2,32 ppm : s : 3H ; 3,15 ppm : s : 3H ; 5,88 ppm : s : 1H ; 6,64 ppm : d : 2H ; 7,12 ppm : d : 2H.

### C) Dichlorhydrate de N,5-diméthyl-1H-indole-2-amine.

On dissout 1,0 g du composé de l'étape précédente dans 20 ml de POCl₃ puis on chauffe à 100°C pendant 2 heures. On refroidit le mélange réactionnel puis on ajoute de l'éther. On filtre le précipité formé et on le lave à l'éther pour obtenir 1,3 g du composé attendu.

RMN DMSO (300 MHz) : 2,31 ppm : s : 3H ; 3,05 ppm : s : 3H ; 4,14 ppm : s : 2H ; 7,07-7,23 ppm : m : 3H ; 10,51 ppm : s : 1H ; 12,37 ppm : d : 1H.

### Préparation 1.3

Chlorhydrate de N,1-diméthyl-5-méthoxy-1*H*-indole-2-amine.

### A) N'-(4-Méthoxyphényl)acétohydrazide.

On dissout 10 g de chlorhydrate de 4-méthoxyphénylhydrazine dans l'eau puis on ajoute de la triéthylamine jusqu'à neutralisation du sel. On extrait à l'AcOEt puis on évapore à sec pour obtenir de 8 g de précipité qui est constitué de 4-méthoxyphényl hydrazine. On dissout ce composé dans 30 ml d'éther puis on ajoute goutte à goutte, une solution de 13 ml d'anhydride acétique dissout dans 30 ml d'éther. On agite 15 minutes à 0°C puis on filtre le précipité blanc formé pour obtenir 7,4 g du composé attendu.

RMN CDCl₃ (200 MHz) : 2,06 ppm : s : 3H ; 3,75 ppm : s : 3H ; 5,65 et 6,03 ppm : 2s : 2H ; 6,6-6,9 ppm : m : 4H.

### B) N,N'-Diméthyl-N'-(4-méthoxyphényl)âcétohydrazide.

On met en suspension 4,3 g de NaH à 60 % dans 30 ml de DMA. On ajoute goutte à goutte, 7,4 g du composé de l'étape précédente dissout dans 20 ml de DMA. Lorsqu'il n'y a plus de dégagement gazeux, on ajoute 10,0 ml d'iodométhane. On agite à température ambiante pendant 1 heure. On verse sur une solution de NH₄Cl saturée puis on extrait à l'AcOEt. On lave plusieurs fois avec une solution de NaCl saturée puis on évapore à sec. On triture le résidu obtenu dans de l'éther de pétrole pour obtenir 8,0 g du composé attendu sous forme d'huile.

RMN CDCl₃ (200 MHz) : 2,19 ppm : s : 3H ; 2,93 ppm : s : 3H ; 3,08 ppm : s : 3H ; 3,80 ppm : s : 3H ; 6,68 ppm : d : 2H ; 6,89 ppm : d : 2H.

### C) Chlorhydrate de N,1-diméthyl-5-méthoxy-1H-indole-2-amine.

On dissout 8,0 g du composé de l'étape précédente dans 30 ml de POCl₃ puis on chauffe à 80°C pendant 2 heures. On refroidit le mélange réactionnel puis on ajoute de l'éther. On filtre le précipité brun formé et on le lave à l'éther pour obtenir 5,3 g du composé attendu, F = 222°C.

RMN DMSO (300 MHz) : 3,06 ppm : s : 3H ; 3,48 ppm : s : 3H ; 3,76 ppm : s : 3H ; 4,26 ppm : s : 2H ; 6,96-7,00 ppm : dd : 1H ; 7,14 ppm : d : 1H ; 7,24 ppm : d : 1H ; 10,08 ppm : s : 1H.

### Préparation 1.4

Chlorhydrate du N,1-diméthyl-5-ehloro-1*H*-indole-2-amine.

### A) N'-(4-Chlorophényl)acétohydrazide.

On dissout 12,5 g de chlorhydrate de la 4-chlorophényl hydrazine dans 100 ml d'eau puis on ajoute de la triéthylamine jusqu'à neutralisation du sel. On extrait à l'AcOEt puis on évapore à sec. On dissout la base dans 100 ml d'éther, on refroidit à 0°C puis on ajoute goutte à goutte, 15 ml d'anhydride acétique. On agite à 0°C pendant 15 minutes. On filtre le précipité blanc formé puis on le lave à l'éther pour obtenir 12,8 g du composé attendu sous forme de poudre blanche.

RMN CDCl₃ (200 MHz) : 2,09 ppm : s : 3H ; 6,68-6,86 ppm : m : 2H ; 7,12-7,30 ppm : m : 2H.

### B) N,N'-Diméthyl-N'-(4-chlorophényl)acétohydrazide.

On met en suspension 7,2 g de NaH à 60 % dans 30 ml de DMA. On ajoute goutte à goutte, 12,8 g d'hydrazine de l'étape précédente dissoute dans 50 ml de DMA puis on agite à température ambiante jusqu'à arrêt du dégagement gazeux. On rajoute goutte à goutte, 17 ml d'iodométhane et on agite à température ambiante pendant 1 heure. On verse sur une solution de NH₄Cl saturée puis on extrait à l'AcOEt. On lave avec une solution de NaCl saturée. On triture le résidu obtenu avec de l'éther de pétrole pour obtenir 10 g du composé attendu sous forme cristalline.

RMN CDCl₃ (200 MHz) : 2,10 ppm : s : 3H ; 2,95 ppm : s : 3H ; 3,10 ppm : s : 3H ; 6,62 ppm : d : 2H ; 7,24 ppm : d : 2H.

### C) Chlorhydrate du N,1-diméthyl-5.chloro-H-indole-2-amine.

On dissout 10 g du composé de l'étape précédente dans 50 ml de POCl₃. On chauffe à reflux pendant 2 heures. On refroidit le milieu réactionnel, on ajoute de l'éther puis on filtre le produit obtenu. On lave plusieurs fois le précipité à l'éther pour obtenir 9,6 g du composé attendu sous forme de poudre.

RMN DMSO (300 MHz) : 3,08 ppm : s : 3H ; 3,52 ppm : s : 3H ; 4,31 ppm : s : 2H ; 7,34 ppm : d : 1H ; 7,48 ppm : d : 1H ; 7,60 ppm : s : 1H ; 10,61 ppm : s : 1H.

### Préparation 1.5

### 1-Méthyl-2-(méthylamino)-1H-indole-5-carboxylate de méthyle.

### A) 4-(2-Acétylhydrazino)benzoate de méthyle.

On dissout 5,5 g de 4-hydrazinobenzoate de méthyle dans 38,2 ml d'AcOH contenant 2,4 g d'acétate de sodium, on chauffe 18 heures à 80°C. On essore le minéral puis on évapore et on reprend par un minimum d'Et₂O. On essore pour obtenir 7,97 g du composé attendu.

### B) 4-(2-Acétyl-1,2-diméthylhydrazino)benzoate de méthyle.

On met en suspension 2,95 g de NaH à 95 % dans 90 ml de DMF et on ajoute goutte à goutte 8,135 g du composé de l'étape précédente en solution dans le minimum de DMF, puis après quelques minutes on ajoute goutte à goutte 9,75 ml d'iodure de méthyle. On agite 1 heure à TA. On verse le milieu sur une solution saturée de NH₄Cl et on extrait par AcOEt. La phase organique est lavée par NaCl, séchée, évaporée pour donner 5,4 g du composé attendu.

### C) 1-Méthyl-2-(méthylamino)-1H-indole-5-carboxylate de méthyle.

On mélange 5,4 g du composé de l'étape précédente et 62 ml d'oxychlorure de phosphore et on chauffe 2 heures et demie à 80°C. On évapore le milieu et on reprend par AcOEt. Le solide formé est essoré, lavé par AcOEt, séché pour donner 4 g du composé attendu.

RMN MeOD (250 MHz) : 3,2 ppm : s : 3H ; 3,6 ppm : s : 3H ; 3,9 ppm : s : 3H ; 7,3-7,4 ppm : m : 2H ; 8,1-8,2 ppm : m : 2H.

En procédant comme décrit ci-dessus, on prépare les composés de formule (II) rassemblés dans le tableau ci-après :

**TABLEAU 1**

| | | | |
|---|---|---|---|
| 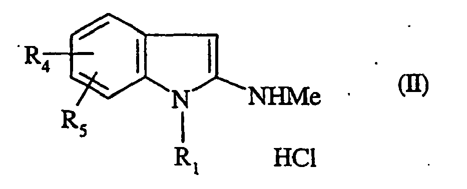 | | | |

| Préparations | R₁ | R₄, R₅ | Caractérisation F°C ou RMN |
|---|---|---|---|
| 1.6 | Me | H | 249°C |
| 1.7 | Me | 7-OMe | 284°C |
| 1.8 | Me | 4-OMe | 103°C |
| 1.9 | Me | 6-OH | 103°C |
| 1.10 | Me | 6-Me | |
| 1.11 | Me | 4,6-diMe | 134°C |
| 1.12 | Me | 5-OMe | 222°C |
| 1.13 | Me | 5-Cl | RMN |
| 1.14 | Me | 6-OMe | 103°C |
| 1.15 | Me | 5-CN | 245°C |
| 1.16 | Me | 5-phényl | |

RMN 1.13 DMSO (300 MHz) : 3,08 ppm : d : 3H ; 3,52 ppm : s : 3H ; 7,34 ppm : d : 1H ; 7,48 ppm : d : 1H ; 7,60 ppm : s : 1H ; 10,61 ppm : s : 1H.

### Préparation 1.17

### Chlorhydrate de 2-(méthylimino)indoline-5-carboxylate d'éthyle.

### A) 4-Hydrazinobenzoate d'éthyle.

On dissout 5,0 g d'acide 4-phénylhydrazine dans 70 ml d'éthanol et 3 ml d'H₂SO₄ concentré. On chauffe à reflux pendant 5 heures, on évapore l'éthanol puis on reprend dans une solution de K₂CO₃ saturée puis on extrait à l'AcOEt. On obtient 5,9 g du composé attendu sous forme de poudre.

RMN CDCl₃ (300 MHz) : 1,38 ppm : t : 3H ; 3,65 ppm : s: 2H ; 4,34 pppm : q : 2H ; 5,57 ppm : s : 1H ; 6,80 ppm : d : 2H ; 7,93 ppm : d : 2H.

### B) 4-(N'-Acétylhydrazino)benzoate d'éthyle.

On dissout 5,9 g du composé de l'étape précédente dans 50 ml d'acide acétique et on ajoute 3,0 g d'acétate de sodium. On chauffe à 80°C pendant 16 heures. On évapore l'acide acétique, on reprend dans de l'eau puis on extrait au CH₂Cl₂. On sèche sur MgSO₄ puis on évapore à sec. On obtient 4,2 g de poudre.

RMN CDCl₃ (300 MHz) : 1,36 ppm : t : 3H ; 2,04 ppm : s: 3H ; 4,32 pppm : q : 2H ; 6,63 ppm : s : 1H ; 6,73 ppm : d (J=8,8) : 2H ; 7,88 ppm : d : 2H ; 8,00 ppm : s : 1H.

### C) 4-(N'-Acétyl-N'-méthylhydrazino)benzoate d'éthyle.

On met en suspension dans 20 ml de DMF, 0,72 g de NaH à 60 %. On refroidit à 0°C, puis on ajoute 4,0 g du composé de l'étape précédente dissous dans 20 ml de DMA. On agite à 0°C pendant 15 minutes puis on ajoute 1,7 ml d'iodométhane et on agite à 0°C pendant 30 minutes. On verse sur une solution de NH₄Cl saturée, on extrait à l'AcOEt, on lave avec une solution de NaCl saturée, on sèche sur MgSO₄ puis on adsorbe sur silice. On purifie par chromatographie sur colonne de silice éluée avec un mélange AcOEt/éther de pétrole (v/v ; 50/50). On obtient 1,8 g d'huile.

RMN CDCl₃ (300 MHz) : 1,38 ppm : t : 3H ; 2,15 ppm : s: 3H ; 3,17 ppm : s : 3H ; 4,35 pppm : q (J=7,1) : 2H ; 6,10 ppm : s : 1H ; 6,71 ppm : d (J=8,8) : 2H ; 7,99 ppm : d : 2H.

### D) Chlorhydrate de 2-(méthylimino)indoline-5-carboxylate d'éthyle.

On dissout 1,5 g du composé de l'étape précédente dans 10 ml de POCl₃. On chauffe à 80°C pendant 2 heures. On refroidit le milieu réactionnel, on ajoute de l'éther, on triture le précipité formé et on le filtre. On lave à l'éther. On obtient 1,4 g de poudre.

RMN CDCl₃ (300 MHz) : 1,31 ppm : t J=7,1): 3H ; 3,07 ppm : d (J=4,7): 3H ; 4,25 ppm : s : 2H ; 4,31 pppm : q : 2H ; 7,58 ppm : d (J=8,8) : 1H ; 7,99 ppm : m : 2H ; 10,97 ppm : s : 1H ; 12,81 ppm : s : 1H.

### Préparation 1.18

Chlorhydrate du 5-bromo-N-méthyl-1*H*-indole-2-amine.

### A) N'-(4-Bromophényl)formic hydrazide.

On dissout 10,0 g de chlorhydrate de 4-bromophénylhydrazine dans 30 ml d'eau. On ajoute 6,2 g de K₂CO₃ et 36 ml de formate de méthyle puis on chauffe à reflux pendant 1 heure puis à température ambiante pendant 12 heures. On filtre le précipité formé et on le lave avec un mélange isopropanol/éther de pétrole (v/v ; 50/50). On obtient 10,5 g du produit attendu.

RMN CDCl₃ (300 MHz) : 6,,73-6,77 ppm : m : 2H ; 7,34-7,41 ppm : m : 2H ; 8,33 ppm : m : 1H.

### B) N'-(4-Bromophényl)-N-méthylacétohydrazide.

On chauffe à reflux une solution de 80 ml de LAH dans du THF. On ajoute 10,5 g du composé de l'étape précédente en suspension dans 60 ml de THF. On chauffe à reflux pendant 15 heures. On refroidit le milieu réactionnel puis on ajoute goutte à goutte 2,3 ml d'eau puis 9,0 ml de NaOH 1N puis encore 10 ml d'H₂O. On filtre les sels sur célite^{®}, on lave à l'AcOEt puis on évapore à sec. On reprend dans 80 ml d'AcOEt puis on ajoute 17 g de K₂CO₃ dissous dans 80 ml d'eau puis 4,0 ml d'anhydride acétique. On agite à température ambiante pendant 1 heure. On sépare les 2 phases, on sèche la phase organique sur MgSO₄ puis on évapore à sec. On ajoute de l'éther de pétrole puis on filtre les cristaux formés. On obtient 9,0 g de produit attendu.

RMN CDCl₃ (300 MHz) : 2,15 ppm : s: 3H ; 3,13 ppm : s : 3H ; 6,57-6,62 ppm : m : 2H ; 7,32-7,40 ppm : m : 2H.

### C) Chlorhydrate du 5-bromo-N-méthyl-1H-indole-2-amine.

On dissout 9,0 g d'hydrazine obtenue à l'étape précédente dans 50 ml de POCl₃ puis on chauffe à 80°C pendant 2 heures. On refroidit le mélange réactionnel puie on ajoute de l'éther. On filtre le précipité formé et on le lave à l'éther. On obtient 8,2 g de poudre.

RMN CDCl₃ (300 MHz) : 4,31 ppm : s : 3H ; 7,14-7,87 ppm : m : 4H ; 10,70 ppm : m : 1H ; 12,62 ppm : s : 1H.

### Préparation 1.19

### 8-Méthyl-1,2,3,4-tétrahydropyrimido[1,2-a]indole.

### A) 1-(4-Méthylphényl)pyrazolidin-3-one.

On dissout 10 g de chlorhydrate de *p*-tolylhydrazine dans 100 ml de CH₂Cl₂ anhydre. On refroidit à 0°C et on ajoute 19 ml de DBU puis 6,5 mg de chlorure de 3-bromopropionyle. On agite à température ambiante pendant 1 heure. On verse le milieu réactionnel sur de l'eau, on extrait au CH₂Cl₂ puis on purifie par chromatographie sue colonne de silice éluée avec un mélange AcOEt/éther de pétrole (v/v ; 50/50). On obtient 1,3 g de cristaux.

RMN CDCl₃ (300 MHz) : 2,35 ppm : s: 3H ; 2,54 ppm : t (J=7,9) : 2H ; 3,89 ppm : q (J=7,9) : 2H ; 6,93-7,16 ppm : m : 4H ; 8,22 ppm : s : 1H.

### B) 1-Acétyl-2-(4-méthylphényl)pyrazolidine.

A 9,6 ml d'une solution de LAH à 1M dans le THF, on ajoute 1,3 g du composé de l'étape précédente dissous dans 20 ml de THF anhydre. On chauffe à reflux pendant 18 heures. On refroidit à température ambiante puis on ajoute 2 ml d'eau et 7 ml de soude 1N puis on filtre les sels sur célite^{®}. On évapore le filtrat et on reprend le résidu dans 20 ml d'AcOEt et on ajoute 2,6 g de K₂CO₃ et 5 ml d'H₂O puis 0,6 ml d'anhydride acétique. On agite à température ambiante pendant 1 heure. On sépare les 2 phases, on sèche sur MgSO₄ la phase organique puis on évapore à sec. On obtient 1,4 g d'huile.

RMN CDCl₃ (300 MHz) : 1,93-2,05 ppm : m: 2H ; 2,07 ppm : s : 3H ; 2,30 ppm : s : 3H ; 3,50 ppm : m : 4H ; 6,83-7,11 ppm : m : 4H.

### C) 8-Méthyl-1,2,3,4-tétrahydropyrimido[1,2-a]indole.

On dissout 1,4 g du composé de l'étape précédente dans 10 ml de POCl₃. On chauffe à 80°C pendant 1 heure 30 minutes. On refroidit le milieu réactionnel, on ajoute de l'éther puis on filtre le précipité formé et on le lave à l'éther. On obtient 1,4 g du composé attendu sous forme de poudre.

RMN DMSO (300 MHz) : 2,06-2,14 ppm : m: 2H ; 2,33 ppm : s : 3H ; 3,50 ppm : m : 2H ; 3,84-3,88 ppm : m : 2H ; 4,15 ppm : s : 2H ; 7,15-7,29 ppm : m : 3H ; 10,84 ppm : s : 1H.

### Préparation des intermédiaires de formule (III) et (IV).

### Préparation 2.1

### 2-(3,5-Difluorophényl)-3-diméthylamino-2-propénoate de méthyle (IV).

### A) 3,5-Difluorophényl acétate de méthyle.

A 0°C, on prépare une solution contenant 25 ml de chlorure d'acétyle dans 250 ml de méthanol, puis, à température ambiante, on dissout 25,5 g d'acide 3,5-difluorophénylacétique dans cette solution et on laisse sous agitation à TA. La réaction est suivie par chromatographie sur couche mince, après disparition du produit de départ, le milieu est évaporé sous pression réduite puis le résidu est dissous dans 250 ml de MTBE. On lave 3 fois par 100 ml d'eau, sèche sur MgSO₄ puis évapore à sec sous pression réduite. On obtient 26,9 g du composé attendu.

### B) 2-(3,5-Difluorophényl)-3-diméthylamino-2-propénoate de méthyle.

On dissout 26,9 g du composé de l'étape précédente dans 61 ml de diméthoxy-N,N-diméthylméthanamine. On chauffe à 135-140°C et on distille le méthanol formé (12 g). On évapore le solvant sous pression réduite et on reprend le résidu par 250 ml de MTBE. On lave 3 fois par 50 ml d'eau puis on sèche sur MgSO₄ et évapore à sec. Le résidu recristallise dans le méthylcyclohexane, on filtre le produit formé puis on le lave 2 fois par 25 ml de méthylcyclohexane pour donner 28 g du composé attendu, F = 97°C.

### Préparation 2.2

### 2-(3,5-Difluorophényl)-3-hydroxy-2-propénoate d'éthyle (III).

### A) 3,5-Difluorophénylacétate d'éthyle.

On dissout 5 g d'acide 3,5-difluorophénylacétique dans 50 ml d'éthanol et 3 ml d'H₂SO₄ concentré et on chauffe à reflux pendant 2 heures. On évapore à sec puis on neutralise avec une solution saturée de K₂CO₃ puis on extrait à l'AcOEt et on évapore pour obtenir 5,0 g du composé attendu sous forme de liquide incolore.

RMN CDCl₃ (200 MHz) : 1,27 ppm : t : 3H ; 3,59 ppm : s : 2H ; 4,18 ppm : q : 2H ; 6,68-6,85 ppm: m : 3H.

### B) 2-(3,5-Difluorophényl)-3-hydroxy-2-propénoate d'éthyle.

On dissout 5,0 g de 3,5-difluorophényl acétate d'éthyle dans 50 ml de formiate d'éthyle. On additionne par petites fractions 2,0 g de NaH à 60 %. On verse sur une solution de HCl 1N puis on extrait à l'AcOEt. On triture le résidu dans de l'éther de pétrole et on filtre le précipité blanc restant puis on évapore le filtrat pour obtenir 3,3 g du composé attendu sous forme d'un liquide.

RMN CDCl₃ (200 MHz) : 1,33 ppm : t : 3H ; 4,34 ppm : q : 2H ; 6,69-7,38 ppm : m : 4H ; 12,16 ppm : m : 1H.

### Préparation 2.3

### 2-(3,5-Diméthylphényl)-3-hydroxy-2-propénoate d'éthyle (III).

On dissout 10 ml de 2-(3,5-diméthylphényl)acétate d'éthyle dans 80 ml de formiate d'éthyle. On additionne par petites fractions 5 g de NaH à 50 % puis on agite à TA pendant 12 heures. On verse sur une solution d'HCl 1N puis on extrait par AcOEt et on évapore pour obtenir le composé attendu, utilisé tel quel à l'étape suivante.

### Préparation 2.4

### 2-(4-méthoxyphényl)-3-hydroxy-2-propénoate d'éthyle (III).

On dissout 8,9 ml de p-méthoxyphénylacétate d'éthyle dans 80 ml de formiate d'éthyle. On additionne par petites fractions, 4,6 g de NaH à 50 %, puis on agite à température ambiante pendant 12 heures. On verse sur une solution de HCl 1N puis on extrait à l'AcOEt. On purifie par chromatographie sur colonne de silice éluée avec un mélange AcOEt/heptane (05/95 ; v/v) pour obtenir 4,0 g du composé attendu sous forme liquide.

RMN CDCl₃ (200 MHz) : 1,30 ppm : t : 3H ; 3,83 ppm : s : 3H ; 4,29 ppm : q : 2H ; 6,89-7,21 ppm: m : 5H.

### Préparation 2.5

### 2-[4-Benzyloxy)phényl]-3-diméthylamino, propénoate de méthyle (IV).

A 5 g de 2-[4-(benzyloxy)phényl]acétate de méthyle dans 5,2 ml de diméthoxy-N,N-diméthylméthanamine, on ajoute 200 µl de tétraméthyléthylènediamine et on agite à 130°C pendant 3 heures. Après refroidissement à TA, on ajoute de l'acétate d'éthyle et 60 ml de chlorure d'ammonium, on agite pendant 5 minutes, on sépare la phase organique et la phase aqueuse est extraite deux fois à l'acétate d'éthyle. Après évaporation du solvat sous pression réduite, puis traitement au charbon actif, on récupère 4,16 g du composé attendu après lavage au pentane du solide.

RMN CDCl₃ (200 MHz) : 2,66 ppm : s : 3H ; 3,62 ppm : s : 3H ; 5,45 ppm : s : 2H ; 6,91 ppm : d : 2H ; 7,14 ppm : d : 2H ; 7,55 ppm : m : 5H ; 7,57 ppm : s : 1H.

### Préparation 2.6

### 2-(3-Bromophényl)-3-hydroxy-2-propénoate d'éthyle (III).

### A) 3-Bromophénylacétate d'éthyle.

On dissout 5 g d'acide 3-bromophénylacétique dans 80 ml d'éthanol, on ajoute 3 ml d'H₂SO₄ concentré puis on chauffe à reflux pendant 2 heures. On évapore l'éthanol, neutralise par une solution saturée de K₂CO₃ puis on extrait par AcOEt et sèche sur MgSO₄. On obtient 5,2 g du composé attendu sous forme liquide.

RMN CDCl₃ (300 MHz) : 1,18 ppm : t : 3H ; 3,50 ppm : s : 2H ; 4,08 ppm : q : 2H ; 7,09-7,37 ppm : m : 4H.

### B) 2-(3-Bromophényl)-3-hydroxy-2-propénoate d'éthyle (III).

On dissout 5,2 g du composé de l'étape précédente dans 70 ml de formiate d'éthyle et on ajoute par petites fractions 1,7 g de NaH à 60 %. On laisse sous agitation à TA pendant 5 heures. On verse sur 100 ml d'HCl 1N puis on extrait par AcOEt, sèche sur MgSO₄ puis évapore à sec. On obtient 5,8 g du composé attendu sous forme d'huile.

RMN CDCl₃ (300 MHz) : 1,9 ppm : t : 3H ; 4,20 ppm : q : 2H ; 7,11-7,42 ppm : m : 5H ; 12,06 ppm : d : 1H.

En procédant selon les Préparations décrites ci-dessus, on obtient les intermédiaires de formule (III) rassemblés dans le tableau ci-après :

**TABLEAU 2**

| | | |
|---|---|---|
| 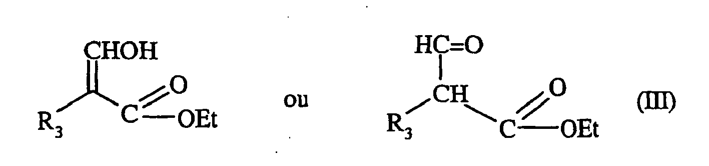 | | |

| Préparations | R₃ | Caractérisation RMN |
|---|---|---|
| 2.7 | 2,4-diCl-phényl | CDCl₃ (300 MHz) : 1,25 ppm : t : 3H ; 4,25 ppm : q : 2H ; 7,13-7,44 ppm : m : 4H ; 12,03 ppm : m : 1H. |
| 2.8 | 3,4-diCl-phényl | CDCl₃ (300 MHz) : 1,29 ppm : t : 3H ; 4,31 ppm : q : 2H ; 7,08-7,42 ppm : m : 4H ; 12,16 ppm : m : 1H. |
| 2.9 | 3-CF₃-phényl | CDCl₃ (200 MHz) : 1,30 ppm : t : 3H ; 4,31 ppm : q : 2H ; 7,31-7,55 ppm : m : 4H ; 12,19 ppm : m : 1H. |
| 2.10 | 3,5-CF₃-phényl | CDCl₃ (200 MHz) : 1,30 ppm : t : 3H ; 4,36 ppm : q : 2H ; 7,30-7,83 ppm : m : 4H ; 12,32 ppm : m : 1H. |
| 2.11 | 1,3-benzodioxazol-5-yl | CDCl₃ (200 MHz) : 1,20 ppm : t : 3H ; 4,33 ppm : q : 2H ; 6 ppm : s : 2H ; 6,7-6,9 ppm : m : 3H ; 7,29 ppm : d : 1H ; 12,06 ppm : d : 1H. |
| 2.12 | 2,5-diOMe-phényl | non purifié |
| 2.13 | 3,4-diOMe-phényl | CDCl₃ (200 MHz) : 1,34 ppm : t : 3H ; 3,91 et 3,92 ppm : 2s : 6H ; 4,33 ppm : q : 2H ; 6,7-7,0 ppm : m : 3H ; 7,32 ppm : d : 1H ; 12,07 ppm : d : 1H. |
| 2.14 | 3,5-diF-phényl | CDCl₃ (200 MHz) : 1,33 ppm : t : 3H ; 4,34 ppm : q : 2H ; 6,69-7,38 ppm : m : 4H ; 12,16 ppm : m : 1H. |
| 2.15 | 2,4-diF-phényl | CDCl₃ (200 MHz) : 1,35 ppm : t : 3H ; 4,24-4,35 ppm : q : 2H ; 6,82-7,30 ppm : m : 4H ; 12,16 ppm : m : 1H. |
| 2.16 | 2,3-diF-phényl | CDCl₃ (200 MHz) : 1,30 ppm : t : 3H ; 4,30 ppm : q : 2H ; 6,96-7,34 ppm : m : 4H ; 12,24 ppm : d : 1H. |
| 2.17 | 3,5-diCl-phényl | CDCl₃ (200 MHz) : 1,26 ppm : t : 3H ; 4,29 ppm : q : 2H ; 7,16-7,49 ppm : m : 4H. |
| 2.18 | 3-F, 5-CF₃-phényl | CDCl₃ (200 MHz) : 1,32 ppm : t : 3H ; 4,31 ppm : q : 2H ; 7,21-7,40 ppm : m : 4H ; 12,26 ppm : m : 1H. |
| 2.19 | 2,4-diF-phényl | CDCl₃ (200 MHz) : 1,35 ppm : t : 3H ; 4,24-4,35 ppm : q (J=7,1) : 2H ; 6-82-7-30 ppm : m : 4H ; 12,16 ppm : m : 1H. |
| 2.20 | 4-SO₂Me-phényl | huile |
| 2.21 | (4-OMe, 3,5 di-tBu)phényl | 1,38 ppm : t (J=7,2) : 3H ; 1,56 ppm : s : 18H ; 3,83 ppm : s : 3H ; 4,41 ppm : q (J=7,2) : 2H ; 7,27-7,44 ppm : m : 3H ; 12,21 : d (J=12,7) : 1H. |
| 2.22 | 3,4,5-triOMe-phényl | CDCl₃ (200 MHz) : 1,36 ppm : t (J=7,1) : 3H ; 3,88-3,89-3,90 ppm : 3s : 9H ; 4,35 ppm : q (J=7,1) : 2H ; 6,53 ppm : s : 2H ; 7,36 ppm : d (J=12,7) : 1H ; 12,12 ppm : d (J=7,1) : 1H. |
| 2.23 | 3,5-diOMe-phényl | CDCl₃ (300 MHz) : 1,32 ppm : t : 3H ; 3,80 ppm : s : 6H ; 4,27-4,34 ppm : q (J=7,2) : 2H ; 6,49 ppm : m : 3H ; 7,34 ppm : d : 1H ; 12,14 ppm : d (J=12,5) : 1H |
| 2.24 | 4-(N₃-phényl) | DMSO (200 MHz) : 1,1 ppm : t : 3H ; 4 ppm : q : 2H ; 7 ppm : d : 2H ; 7,3 ppm : d : 2H ; 7,8 ppm : s : 1H ; 11 ppm : s : 1H |
| 2.25 | 2,4-diOMe-phényl | |
| 2.26 | 3-(N₃-phényl) | DMSO (200 MHz) : 1,2 ppm : t : 3H ; 4,1 ppm : q : 2H ; 7 ppm : s : 1H ; 7,05 ppm : d : 1H ; 7,2 ppm : d : 1H; 7,35 ppm : m : 2H; 7,9 ppm : s : 1H. |
| 2.27 | 2-Cl-4-F-phényl | CDCl₃ (300 MHz) : 1,16 ppm : t (J=7,2) : 3H ; 4,13 ppm : q (J=7,2) : 2H ; 6,89-7,90 ppm : m : 4H ; 11,90 ppm : d (J=12,7) : 1H |

### Préparation 2.28

### 2-(6-Chloro-1,3-benzodioxol-5-yl)-3-hydroxy acrylate d'éthyle.

### A) 5-Bromométhyl-6-chloro-1,3-benzodioxole.

On dissout 2,5 g de 6-chloropipéronylalcool dans 80 ml d'éther éthylique. On refroidit à 0°C puis on ajoute 1,9 ml de PBr₃. On agite à température ambiante pendant 18 heures. On verse sur de la glace puis on extrait à l'AcOEt. On lave avec une solution de NaCl saturée. On obtient 3,3 g de poudre.

RMN CDCl₃ (300 MHz) : 4,47 ppm : s : 2H ; 5,92 ppm : s : 2H ; 6,77 ppm : s : 1H ; 6,88 ppm : s : 1H.

### B) (6-Chloro-1,3-benzodioxol-5-yl)acétonitrile.

On dissout 3,3 g du composé de l'étape précédente dans 70 ml d'éthanol et 15 ml d'eau. On ajoute 1,8 g de KCN puis on chauffe à reflux pendant 5 heures. On évapore l'éthanol, on reprend dans de l'eau puis on extrait à l'AcOEt. On sèche sur MgSO₄ puis on évapore à sec. On obtient 2,4 g d'huile.

RMN CDCl₃ (300 MHz) : 3,75 ppm : s : 2H ; 6,02 ppm : s : 2H ; 6,88 ppm : s : 1H ; 6,95 ppm : s : 1H.

### C) (6-Chloro-1,3-benzodioxol-5-yl)acétate d'éthyle.

On dissout 2,4 g de composé de l'étape précédente dans 80 ml d'éthanol et 4 ml d'H₂SO₄ concentré. On chauffe à reflux pendant 48 heures. On évapore l'éthanol, on reprend dans de l'eau puis on extrait à l'AcOEt. On lave avec une solution de NaCl saturée, on sèche sur MgSO₄ puis on évapore à sec. On obtient 2,9 g d'huile contenant 20 % environ de produit de départ.

RMN CDCl₃ (300 MHz) : 1,26 ppm : t (J=6,9) : 3H ; 3,68 ppm : s : 2H ; 4,15-4,23 ppm : q (J=6,9) : 2H ; 5,98 ppm : s : 2H ; 6,77 ppm : s : 1H ; 6,87 ppm : s : 1H.

### D) 2-(6-Chloro-1,3-benzodioxol-5-yl)-3-hydroxy acrylate d'éthyle.

On dissout 2,9 g de composé de l'étape précédente dans 60 ml de formate d'éthyle. On ajoute 1,0 g de NaH à 60 % puis on agite à température ambiante pendant 5 heures. On verse 100 ml d'une solution de HCl 1N puis on extrait à l'AcOEt, on sèche sur MgSO₄ et on évapore à sec. On obtient 3,2 g d'huile utilisée telle quelle pour la suite.

### Préparation 2.29

### 2-(3-Hydroxyphényl)-3-diméthylaminoacrylate d'éthyle.

### A) (3-Hydroxyphényl)acétate de méthyle.

On dissout 10 g d'acide 3-hydroxyphénylacétique dans 60 ml de méthanol et 2,5 ml d'acide sulfurique. On chauffe à reflux pendant 2 heures. On revient à température ambiante et évapore le méthanol. On reprend le résidu dans une solution de K₂CO₃ saturée. On extrait à l'AcOEt. On sèche la phase organique sur MgSO₄, on filtre et on évapore à sec. On obtient 11,1 g d'huile.

RMN DMSO (300 MHz) : 3,57 ppm : s : 3H ; 3,61 ppm : s : 2H ; 6,66 ppm : m : 3H ; 7,09 ppm : m : 1H ; 9,42 ppm : s : 1H.

### B) (3-benzyloxyphényl)acétate d'éthyle.

On dissout 3 g du composé de l'étape précédente dans 13 ml d'éthanol. On ajoute 3,75 g de K₂CO₃, 3,12 ml de chlorure de benzyle et une pointe de spatule de nBu₄NI. On chauffe à reflux pendant 6 heures. On revient à température ambiante, on filtre sur K₂CO₃, on évapore à sec. On reprend dans l'AcOEt et on lave à l'eau. On obtient 5 g d'huile.

RMN DMSO (300 MHz) : 1,19 ppm : t : 3H ; 3,64 ppm : s : 2H ; 5,08 ppm : s : 2H ; 6,85-6,98 ppm : m : 2H ; 7,22-7,51 ppm : m : 7H.

### C) 2-(3-Hydroxyphényl)-3-diméthylaminoacrylate d'éthyle.

On dissout 5 g du composé de l'étape précédente dans 8 ml de diméthylformamide diméthylacétal (DMFDMA). On chauffe à 135°C pendant 24 heures en ajoutant 1 ml de DMFDMA toutes les 3 heures environ. On évapore à sec. On obtient 6 g d'huile.

En procédant selon la Préparation 2.1, on obtient les intermédiaires de formule (IV) rassemblés dans le tableau ci-après :

**TABLEAU 3**

| | | |
|---|---|---|
| 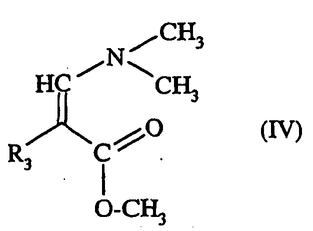 | | |

| Préparations | R₃ | Caractérisation RMN |
|---|---|---|
| 3.1 | 4-NMe₂-phényl | |
| 3.2 | 2,6-diCl-phényl | DMSO (200 MHz) : 2,8 ppm : s : 6H ; 3,5 ppm : s : 3H ; 7,3-7,55 ppm : m : 3H ; 7,6 ppm : s : 1H. |
| 3.3 | 3-Br, 4-OMe-phényl | 125°C |
| 3.4 | 2,4-diCl-phényl | DMSO (200 MHz) : 2,6 ppm : s : 6H ; 3,4 ppm : s : 3H ; 7,1-7,3 ppm : m : 2H ; 7,4-7,5 ppm : m : 2H. |
| 3.5 | 2-Br-4,5-diOMe-phényl | 115°C |
| 3.6 | 2-Cl-4,5-diOMe-phényl | DMSO (200 MHz) : 2,6 ppm : s : 6H ; 3,4 ppm : s : 3H ; 3,6 ppm : s : 3H ; 3,65 ppm : s : 3H ; 6,65 ppm : s : 1H ; 6,85 ppm : s : 1H ; 7,4 ppm : s : 1H. |
| 3.7 | 3-CN-4-OMe-phényl | 125°C |
| 3.8 | 2,4-diMe-phényl | - |
| 3.9 | 3,4-diMe-phényl | - |
| 3.10 | 4-OBn-phényl | DMSO/TFA (200 Mhz) : 2,6 ppm : s : 6H ; 3,4 ppm : s : 3H ; 5 ppm : s : 2H ; 6,8-7 ppm : m : 4H ; 7,1-7,25 ppm : m : 6H. |
| 3.11 | 4-(OCH₂COOMe)-phényl | DMSO (300 MHz) : 2,64 ppm : s : 6H ; 3,50 ppm : s : 3H ; 3,70 ppm : s : 3H ; 4,75 ppm : s : 2H ; 6,64-7,48 ppm : m : 4H ; 7,95 ppm : s : 1H. |
| 3.12 | 3,5-diOMe-phényl | CDCl₃ (300 MHz) : 2,30 ppm : s : 6H ; 2,68 ppm : s : |
| | | 6H ; 3,64 ppm : s : 3H ; 6,81-6,86 ppm : m : 3H ; 7,54 ppm : s : 1H |

### Préparation 3.13

### 2-(4-Bromo-2-chlorophényl)-3-diméthylaminoacrylate d'éthyle.

### A) 4-Bromo-1-bromométhyl-2-chlorobenzène.

On dissout 5,0 g de 2-chloro-4-bromotoluène dans 120 ml de CCl₄. On ajoute 4,3 g de NBS et 1,6 g de AIBN. On chauffe à reflux pendant 15 heures, on ajoute de l'eau, on sépare les deux phases puis on extrait au CH₂Cl₂. On purifie par chromatographie sur colonne de silice éluée avec de l'éther de pétrole. On obtient 3,8 g de liquide.

CDCl₃ (300 MHz) : 4,48 ppm : s : 2H ; 7,23-7,36 ppm : m : 2H ; 7,51 ppm : s : 1H.

### B) (4-Bromo-2-chlorophényl)acétonitrile.

On dissout 3,8 g du compose de l'étape précédente dans 70 ml d'éthanol et 15 ml d'H₂O. On ajoute 1,7 g de KCN puis on chauffe à reflux pendant 5 heures. On évapore l'éthanol, on reprend dans de l'eau puis on extrait à l'AcOEt. On obtient 2,5 g d'huile.

CDCl₃ (300 MHz) : 3,79 ppm : s : 2H ; 7,35-7,49 ppm : m : 2H ; 7,59 ppm : s : 1H.

### C) (4-Bromo-2-chlorophényl)acetate d'éthyle.

On dissout 2,5 g du composé de l'étape précédente dans 80 ml d'éthanol et 4 ml d'H₂SO₄ concentré. On chauffe à reflux pendant 4 jours. On évapore l'éthanol, on reprend dans une solution de K₂CO₃ saturée, on sèche sur MgSO₄ puis on évapore à sec. On obtient 2,5 g de liquide.

CDCl₃ (300 MHz) : 1,28 ppm : t (J=7,1) : 3H ; 3,73 ppm : s : 2H ; 4,18 ppm : q (J=7,1) : 2H ; 7,18 ppm : m : 1H ; 7,38 ppm : m : 1H ; 7,56 ppm : s : 1H.

### D) 2-(4-Bromo-2-chlorophényl)-3-diméthylaminoacrylate d'éthyle.

On dissout 2,8 g du composé de l'étape précédente dans 3,1 g de réactif de Bredereck. On chauffe à 100°C pendant 15 heures, on évapore l'excès de réactif. On obtient 2,9 g d'huile.

CDCl₃ (300 MHz) : 1,18 ppm : t (J=7,1) : 3H ; 2,72 ppm : s : 6H ; 4,09 ppm : q (J=7,1) : 2H ; 7,15 ppm : m : 1H ; 7,35 ppm : m : 1H ; 7,54 ppm : m : 1H ; 7,60 ppm : s : 1H.

### Exemple 1 : composé 6

### 3-(3,5-Difluorophényl)-1,6,9-triméthyl-1,9-dihydro-2H-pyrido[2,3-b]indol-2-one.

On mélange 25 g du composé de la Préparation 2.1 et 22,5 g du composé de la Préparation 1.1 dans 75 ml d'acide acétique et on chauffe à 40°C pendant 24 heures puis à 60-65°C pendant 4 heures. On refroidit le milieu réactionnel à TA puis on le verse sur 275 ml d'eau. Le précipité formé est récupéré, lavé par 50 ml d'eau puis recristallisé dans 250 ml de MiBK, l'eau résiduelle est éliminée par distillation azéotropique à pression atmosphérique. Le produit obtenu est lavé 2 fois par 25 ml de MiBK puis séché à 40-45°C sous pression réduite pendant 24 heures pour donner 20,33 g du composé attendu après cristallisation dans MiBK (20,33 g), F = 240°C.

RMN DMSO (200 MHz) : 2,51 ppm : s : 3H ; 4,05 ppm : s : 6H ; 6,69-8,08 ppm : m : 7H.

### Exemple 2 : composé 22

### 3-(3,5-Difluorophényl)-1,6-diméthyl-1,9-dihydro-2H-pyrido[2,3-b]indol-2-one.

On dissout 1,5 g de chlorhydrate de la Préparation 1.2 dans 50 ml de pyridine. On ajoute 1,9 g du composé de la Préparation 2.2 et on chauffe à 70°C pendant 20 heures. On évapore à sec puis on reprend dans de l'eau et on extrait au CH₂Cl₂. On purifie par chromatographie sur colonne de silice éluée avec un mélange AcOEt/heptane (50/50 ; v/v) pour obtenir 300 mg du composé attendu sous forme de poudre, F = 189°C (décomposition).

RMN DMSO (300 MHz) : 2,51 ppm : s : 3H ; 3,70 ppm : s : 3H ; 7,04-8.68 ppm : m : 7H ; 11,98 ppm : s : 1H.

### Exemple 3 : composé 16

### 1,6-Diméthyl-3-(3,5-diméthylphényl)-1,9-dihydro-2H-pyrido [2,3-b] indol-2-one.

On dissout 0,8 g du composé de la Préparation 1.2 obtenu dans 50 ml de pyridine. On ajoute 0,8 g de composé de la Préparation 2.3 et on chauffe à 80°C pendant 20 heures. On évapore à sec puis on reprend dans de l'eau et on extrait au CH₂Cl₂. On purifie par chromatographie sur colonne de silice éluée avec un mélange AcOEt/heptane (50/50 ; v/v) pour obtenir 500 mg de composé attendu, après recristallisation dans l'isopropanol.

RMN DMSO (200 MHz) : 2,23 ppm : s : 6H ; 2,49 ppm : s : 3H ; 3,73 ppm : s : 3H ; 6,84-8,08 ppm : m : 7H ; 9,98 ppm : s : 1H.

### Exemple 4 : composé 20

### 6-Méthoxy-1,9-diméthyl-3-(3,5-difluorophényl-1,9-dihydro-2H-pyrido[2,3-b] indol-2-one.

On dissout 2,5 g du composé de la Préparation 1.3 dans 70 ml de pyridine. On ajoute 3,3 g du composé de la Préparation 2.2 et on chauffe à 100°C pendant 20 heures. On évapore à sec puis on reprend dans de l'eau et de l'AcOEt. On filtre le précipité restant. On recristallise dans de l'isopropanol pour obtenir 1,35 g du composé attendu, F = 189°C.

RMN CDCl₃ (200 MHz) : 3,91 ppm : s : 3H ; 4,04 ppm : s : 6H ; 6,68-8,05 ppm : m : 7H.

### Exemple 5 : composé 1

### 3-(3,5-Diméthylphényl)-1,6,9-triméthyl-1,9-dihydro-2H-pyrido[2,3-b]indol-2-one.

Ce composé est préparé à partir des composés des Préparations 1.1 et 2.3, F = 210°C.

RMN CDCl₃ (200 MHz) : 2,42 ppm : s: 6H ; 2,54 ppm : s : 3H ; 4,07 ppm : s : 6H ; 7,00-8,08 ppm : m : 7H.

### Exemple 6 : composé 2

### 3-(2,4-Dichlorophényl)-1,6,9-triméthyl-1,9-dihydro-2H-pyrido[2,3-b]indol-2-one.

Ce composé est préparé à partir des composés des Préparations 1.1 et 2.7, F = 170°C.

RMN CDCl₃ (300 MHz) : 2,51 ppm : s : 3H ; 4,09 ppm : s : 3H ; 4,11 ppm : s : 3H ; 7,15-8,00 ppm : m : 7H.

### Exemple 7 : composé 21

### 3-(2,4-Dichlorophényl)-6-méthoxy-1,9-diméthyl-1,9-dihydro-2H-pyrido[2,3-b] indol-2-one.

Ce composé est préparé à partir des composés des Préparations 1.3 et 2.7, F = 130°C.

RMN CDCl₃ (300 MHz) : 3,90 ppm : s : 3H ; 4,07 ppm : s : 3H ; 4,09 ppm : s : 3H ; 6,93-7,98 ppm : m : 7H.

### Exemple 8 : composé 32

### 3-(2,4-Dichlorophényl)-1,5,7,9-tétraméthyl-1,9-dihydro-2H-pyrido[2,3-b]indol-2-one.

Ce composé est préparé à partir des composés des Préparations 1.11 et 2.7, F = 204°C

RMN CDCl₃ (300 MHz) : 2,51 ppm : s : 3H ; 2,68 ppm : s : 3H ; 4,10 ppm : s : 6H ; 6,91 ppm : s : 1H ; 7,05 ppm : s : 1H ; 7,31 ppm : dd : 1H ; 7,44 ppm : d : 1H ; 7,52 ppm : d : 1H ; 8,11 ppm : s : 1H.

### Exemple 9 : composé 66

### 3-(4-Hydroxyphényl)-1,6,9-triméthyl-1,9-dihydro-2H-pyrido[2,3-b]indolone.

### A) 3-(4-Méthoxyphényl)-1,6,9-triméthyl-1,9-dihydro-2H-pyrido[2,3-b]indolone.

On dissout 2,0 g de chlorhydrate obtenu à la Préparation 1.1 dans 50 ml de pyridine. On rajoute 2,1 g de formyl ester obtenu à la Préparation 2.4 et on chauffe à 80°C pendant 20 heures. On évapore à sec puis on extrait au CH₂Cl₂ et on lave à l'eau. On purifie par chromatographie sur colonne de silice éluée avec un mélange AcOEt/heptane/CH₂Cl₂ (50/50/50 ; v/v/v) pour obtenir 0,5 g du composé attendu sous forme de poudre.

RMN DMSO (200 MHz) : 2,50 ppm : s : 3H ; 3,86 ppm : s : 3H ; 4,05 ppm : s : 6H ; 6,96-8,04 ppm : m : 8H.

### B) 3-(4-Hydroxyphényl)-1,6,9-triméthyl-1,9-dihydro-2H-pyrido[2,3-b]indolone.

A 5,36 g du composé de l'étape précédente, dissous dans 60 ml de DCM, on ajoute sous agitation et à -78°C, 18 ml de BBr₃ en solution dans le DCM. On laisse revenir le mélange à température ambiante. Ce mélange est ensuite agité pendant 24 heures à cette température. Le milieu réactionnel est dilué avec un mélange de DCM et de MeOH. La solution est évaporée sous pression réduite. Le brut est repris dans du DCM et adsorbé sur 16 g de silice et chromatographié sur colonne de silice avec un mélange (97/3 ; v/v) puis (95/5 ; v/v) jusqu'à (50/50 ; v/v) de DCM/MeOH. On récupère ainsi un solide que l'on suspend dans un mélange de DCM/MeOH. On refroidit et on filtre la suspension obtenue. Le précipité est alors collecté. On récupère ainsi 4 g de composé attendu contenant 4 % de composé de départ, F > 280°C.

RMN DMSO (200 MHz) : 2,3 ppm : s : 3H ; 3,9 ppm : s : 3H ; 4 ppm : s : 3H ; 6,6 ppm : d : 2H ; 7 ppm : d : 1H ; 7,3 ppm : d : 1H ; 7,5 ppm : d : 2H ; 7,6 ppm : s : 1H ; 8,1 ppm : s : 1H.

### Exemple 10 : composé 68

### 1,6-Diméthyl-3-(4-hydroxyphényl)-1,9-dihydro-2H-pyrido[2,3-b]indolone.

### A) 1,6-Diméthyl-3-(4-benzyloxyphényl)-1,9-dihydro-2H-pyrido[2,3-b]indolone.

On mélange 1 g de N,5-diméthyl-1*H*-indole-2-amine (Préparation 1.2) avec 1,4 g du composé de la Préparation 2.5 et 10 ml d'acide acétique, puis on chauffe à 100°C pendant 18 heures. On évapore à sec sous vide, puis on reprend par 20 ml de CH₂Cl₂ et 5 ml d'H₂O. On amène le pH à 7 par NaOH 1N sous agitation puis on décante, on lave la phase organique par NaCl puis on sèche et évapore à sec. On reprend le résidu par 10 ml d'Et₂O, essore puis lave la phase organique et sèche.

### B) 1,6-Diméthyl-3-(4-hydroxyphényl)-1,9-dihydro-2H-pyrido[2,3-b]indolone.

On mélange 0,800 g du composé de l'étape précédente et 50 ml de TFA. On chauffe 1 heure 30 minutes à 75°C. On évapore à sec sous vide puis on reprend par 15 ml d'Et₂O et sèche, F = 186°C.

RMN DMSO (300 MHz) : 2,6 ppm : s : 3H ; 3,8 ppm : s : 3H ; 6,8 ppm : d : 2H ; 7,1 ppm : d : 1H ; 7,4 ppm : d : 1H ; 7,6 ppm : d : 2H ; 7,8 ppm : s : 1H ; 8,2 ppm : s : 1H ; 11,9 ppm: s: 1H.

### Exempte 11 : composé 52

### 4-(1,6,9-triméthyl-2-oxo-2,9-dihydro-1H-pyrido[2,3-b]indol-3-yl)benzonitrile.

### A) 3-(3-Bromophényl)-1,6,9-triméthyl-1,9-dihydro-2H-pyrido[2,3-b]indol-2-one.

On dissout 2,5 g du composé de la Préparation 1.1 dans 40 ml d'acide acétique et 60 ml de pyridine ; on ajoute 3,5 g du composé de la Préparation 2.6 puis on chauffe à 100°C pendant 15 heures. On verse le milieu réactionnel dans 200 ml d'eau et on filtre le précipité formé. Celui-ci est repris par CH₂Cl₂ puis lavé par une solution saturée de NaCl, sèché sur MgSO₄ et évaporé à sec. On reprend le précipité dans un mélange AcOEt/cyclohexane (20/8 ; v/v) puis on le filtre. On obtient 20 g du composé attendu, F = 215-216°C.

### B) 4-(1,6,9-triméthyl-2-oxo-2,9-dihydro-1H-pyrido[2,3-b]indol-3-yl)benzonitrile.

On dissout 3g du composé obtenu à l'étape précédente dans 50 ml de 1-méthyl-2-pyrrolidinone, on ajoute 1,4 g de CuCN puis on chauffe à 200°C pendant 4 heures. On verse le milieu réactionnel sur 100 ml de CH₂Cl₂ puis on filtre le précipité formé. On lave le filtrat avec une solution de HCl 1N puis on sèche sur MgSO₄. Le produit obtenu est purifié par chromatographie sur silice en éluant par AcOEt/CH₂Cl₂ (50/50 ; v/v) puis AcOEt/MeOH/NH₃ (90/10/1 ; v/v/v). on recueille 2,2 g du composé attendu sous forme de poudre.

RMN CDCl₃ (300 MHz) : 2,52 ppm : s : 3H ; 4,09 ppm : s : 3H ; 4,12 ppm : s : 3H ; 7,16 ppm : m : 8H.

### Exemple 12 : composé 53

### 3-(4-(aminométhyl)phényl)-1,6,9-triméthyl-1,9-dihydro-2H-pyrido[2,3-b]indol-2-one.

On dissout 50 mg de soude dans 11 ml d'éthanol et on ajoute 0,2 g du composé préparé à l'exemple précédent puis environ 100 mg de Ni de Raney et on hydrogène pendant 24 heures sous 50 psi. On filtre le catalyseur sur célite^{®} puis on rince au méthanol et on évapore à sec. On reprend le résidu dans une solution de HCl 1N puis on extrait les impuretés avec AcOEt. On alcalinise à pH = 9 par une solution de K₂CO₃ puis on extrait par AcOEt pour obtenir 90 mg du composé attendu sous forme de poudre.

RMN CDCl₃ (300 MHz) : 2,51 ppm : s : 3H ; 3,91 ppm : s : 2H ; 4,08 ppm : s : 6H ; 7,13-8,09 ppm : m : 8H.

### Exemple 13 : composé 84

### (3-(2,4-Dichlorophényl)-1,6-diméthyl-2-oxo-1,2-dihydro-9H-pyrido[2,3-b]indol-9-yl) acétate de méthyle.

On dissout 1 g de 3-(2,4-dichlorophényl)-1,6-diméthyl-1,9-dihydro[2,3-b]indol-2-one (composé 46) dans 10 ml de DMF, on ajoute 0,143 g de NaH à 95 % et, après 30 minutes sous agitation, 0,4 ml de bromacétate de méthyle. Après 1 heure sous agitation à TA, on évapore le milieu réactionnel puis on reprend le résidu par CH₂Cl₂ et on lave par une solution de NaHCO₃ puis une solution de NaCl. On sèche puis évapore pour obtenir 0,98 g du produit attendu.

### Exemple 14 : composé 85

### 2-(3-(2,4-Dichlorophényl)-1,6-diméthyl-2-oxo-1,2-dihydro-9H-pyrido[2,3-b] indol-9-yl-N-méthylacétamide.

On mélange 0,3 g du l'ester obtenu à l'exemple précédent et 30 ml de méthylamine à 33 % dans EtOH. Après 5 heures sous agitation à TA, on évapore le milieu réactionnel, on reprend le résidu par Et₂O puis on essore et sèche pour obtenir 0,260 g du composé attendu.

### Exemple 15 : composé 81

### 3-(3-Hydroxyméthylphényl)-1,6-diméthyl-1,9-dihydro-2H-pyrido[2,3-b]indol-2-one,

### A) 3-(1,6-diméthyl-2-oxo-2,9-dihydro-1H-pyrido[2,3-b]indol-3-yl)benzaldéhyde.

On mélange 450 mg de 3-(3-cyanophényl)-1,6-diméthyl-1,9-dihydro-2*H-*pyrido[2,3-b]indol-2-one, 10 ml d'AcOH, 20 ml de pyridine, 2,6 g d'hypophosphite de sodium et 434 mg de Ni de Raney puis on chauffe 4 heures à 60°C. On essore le milieu réactionnel et on évapore le filtrat ; le résidu est repris dans 50 ml d'AcOEt/CH₂Cl₂ (1/1 ; v/v), on lave à l'eau, sèche et évapore pour obtenir le composé attendu, F = 280°C.

### B) 3-(3-Hydroxyméthylphényl)-1,6-diméthyl-1,9-dihydro-2H-pyrido[2,3-b]indol-2-one.

On place 280 mg du composé obtenu à l'étape A dans 10 ml de CH₂Cl₂ et un minimun d'AcOH pour dissoudre le composé et on ajoute 375 mg de NaBH(OAc)₃ puis on laisse 18 heures sous agitation à TA. On évapore le milieu réactionnel, reprend le résidu par AcOEt puis on essore pour obtenir 200 mg du composé attendu.

### Exemple 16 : composé 80

### 1,6-Diméthyl-3-(3-((méthylamino)méthyl)phényl)-1,9-dihydro-2H-pyrido[2,3-b] indol-2-one.

On place 280 mg du composé de l'étape A de l'exemple précédent dans 10 ml de CH₂Cl₂ et un minimum d'AcOH pour dissoudre le composé puis on ajoute 375 mg de NaBH(OAc)₃ et 0,064 ml de méthylamine. Après 18 heures sous agitation à TA, on extrait 3 fois par 10 ml d'eau puis on extrait la phase aqueuse par AcOEt. Après évaporation on obtient 15 mg du composé attendu.

### Exemple 17 : composé 83

### 3-(1,6-Diméthyl-2-oxo-2,9-dihydro-1H-pyrido[2,3-b]indol-3-yl)benzaldéhyde oxime.

On dissout 210 mg du composé de l'exemple 15, étape A, dans 5 ml de MeOH et on ajoute 46 mg de chlorhydrate d'hydroxylamine en solution dans le minimum d'eau et on laisse sous agitation 2 heures à TA. On évapore à sec puis le résidu est chromatographié sur silice en éluant par AcOEt/CH₂Cl₂ (2/8 ; v/v). On obtient 74 mg du composé attendu.

### Exemple 18 : composé 110

### 1,6-Diméthyl-3-(3-méthoxycarbonylphényl)-1,9-dihydro-2H-pyrido[2,3-b]indol-2-one.

### A) 3-(3-Bromophényl)-1,6-diméthyl)-1,9-dihydro-2H-pyrido[2,3-b]indol-2-one.

Ce composé est préparé selon les méthodes habituelles par action du N,1,5-triméthyl-1*H*-indole-2-amine sur le 2-(3-bromophényl)-3-hydroxy-2-propénoate d'éthyle.

RMN DMSO (200 MHz) : 2,42 ppm : s : 3H ; 3,69 ppm : s : 3H ; 7,04 ppm : d : 1H ; 7,35 ppm : d : 1H ; 7,05-7,08 ppm : m : 5H ; 8,30 ppm : s : 1H ; 11,91 ppm : s : 1H.

### B) 1,6-Diméthyl-3-(3-méthoxycarbonylphényl)-1,9-dihydro-2H-pyrido[2,3-b]indol-2-one.

On dissout 500 mg du composé de l'étape précédente, ainsi que 140 mg de 1,3-bis(diphénylphosphino)propane dans 20 ml de MeOH anhydre. On ajoute 1,9 ml de triéthylamine à 99,9 %, 15 ml de DMSO anhydre puis 60 mg de Pd(OAc)₂. On fait buller du CO dans le milieu réactionnel pendant 20 minutes et on chauffe à 75°C pendant une nuit sous atmosphère de CO. On laisse revenir à température ambiante. On verse le milieu réactionnel sur 200 ml d'eau puis on extrait à l'AcOEt. On sèche la phase organique sur MgSO₄, on filtre et on évapore à sec. On purifie sur colonne de silice éluée avec un mélange AcOEt/éther de pétrole (v/v ; 75/25). On triture le précipité obtenu avec un mélange AcOEt/éther de pétrole. On obtient 260 mg d'une poudre.

RMN DMSO (300 MHz) : 2,43 ppm : s : 3H ; 3,70 ppm : s : 3H ; 3,88 ppm : s : 3H ; 7,05 ppm : m : 1H ; 7,35 ppm : d (J=8) : 1H ; 7,54 ppm : m : 1H ; 7,74 ppm : m : 1H ; 7,86 ppm : m : 1H ; 8,02 ppm : m : 1H ; 8,43 ppm : m : 1H ; 11,94 ppm : s : 1H.

### Exemple 19 : composé 111

### 3-(4-Aminophényl)-1,6-diméthyl-1,9-dihydro-2H-pyrido[2,3-b]indol-2-one.

### A) 3-(4-Bromophényl)-1,6-diméthyl)-1,9-dihydro-2H-pyrido[2,3-b]indol-2-one.

Ce composé est préparé selon les méthodes habituelles par action du N,1,5-triméthyl-1*H*-indole-2-amine sur le 2-(4-bromophényl)-3-hydroxy-2-propénoate d'éthyle.

RMN DMSO (300 MHz) : 2,35 ppm : s : 3H ; 3,61 ppm : s : 3H ; 3,97 ppm : d : 1H ; 7,03 ppm : d : 1H ; 7,45-7,80 ppm : m : 5H ; 8,32 ppm : s : 1H ; 11,85 ppm : s : 1H.

### B) 3-(4-Aminophényl)-1,6-diméthyl-1,9-dihydro-2H-pyrido[2,3-b]indol-2-one.

On dégaze pendant 10 minures, 10 ml de THF, on ajoute sous argon 9,2 mg de Pd₂(dba)₃ puis 370 mg de composé de l'étape précédente puis 9,4 mg de ligand (2-dicyclohexylphosphino-2'-(N,N-diméthylamino)biphényl et enfin 2,2 ml d'une solution de LiN(TMS)₂ à 1M dans le THF. On agite dans un tube scellé à 90°C pendant 20 heures. On refroidit le milieu réactionnel puis on ajoute 10 ml d'une solution de HCl 1N puis on agite à température ambiante pendant 10 minutes. On sépare les 2 phases, on lave la phase aqueuse avec une solution d'AcOEt puis on alcanilise avec une solution de K₂CO₃ saturée. On filtre le précipité formé. On obtient 90 mg.

RMN DMSO (300 MHz) : 2,40 ppm : s : 3H ; 3,65 ppm : s : 3H ; 4,99 ppm : s : 2H ; 6,57 ppm : m : 2H ; 6,94 ppm : d (J=8,1) : 1H ; 7,29 ppm : d (J=8,1) : 1H ; 7,43 ppm : m : 2H ; 7,60 ppm : s : 1H ; 8,07 ppm : s : 1H.

### Exemple 20 : composé 121

### 1,6-Diméthyl-1,9-dihydro-3-(phénylaminophényl)-2H-pyrido[2,3-b]indol-2-one.

On met sous argon 13 mg de Pd₂(dba)₃, 500 mg du composé de l'Exemple 18 étape A, 11 mg de ligand (2-dicyclohexylphosphino-2'-(N,N-diméthylamino)biphényl, 3 ml d'une solution de LiN(TMS)₂ à 1M dans le THF, 127 mg d'aniline, puis 10 ml de dioxane anhydre. On agite dans un tube scellé à 65°C pendant 24 heures. On fait revenir à température ambiante, puis on ajoute de l'AcOEt. On lave la phase organique avec une solution de NaCl saturée. On purifie sur colonne de silice éluée avec un mélange AcOEt/éther de pétrole (v/v ; 50/50) puis (v/v ; 75/25). On triture le précipité obtenu dans l'éther. On obtient 55 mg du composé attendu sous forme d'un précipité, F = 188-190°C.

RMN DMSO (300 MHz) : 2,42 ppm : s : 3H ; 3,68 ppm : s : 3H ; 6,77-8,28 ppm : m : 14H ; 12,00 ppm : s : 1H.

### Exemple 21: composé 147

### 3-(2,4-Dicyanophényl)-1,6-diméthyl-1,9-dihydro-2H-pyrido[2,3-b]indol-2-one.

On dissout 0,6 g de 3-(2-chloro-4-bromophényl)-1,6-diméthyl-1,9-dihydro-2*H-*pyrido[2,3-b]indol-2-one dans 15 ml de NMP. On ajoute 0,27 g de CuCN puis on chauffe à reflux pendant 5 heures. On verse le milieu réactionnel sur du CH₂Cl₂ et on lave avec une solution de NaCl saturée. On purifie par chromatographie sur colonne de silice éluée avec un mélange AcOEt/éther de pétrole (v/v ; 75/25) puis AcOEt pur. Les fractions récupérées sont lavés avec une solution de HCl 1N pour ôter un résidu de NMP. On obtient 50 mg du composé attendu sous forme de poudre.

RMN DMSO (300 MHz) : 2,27 ppm : s : 3H ; 3,70 ppm : s : 3H ; 7,08 ppm : d (J=8,1) : 1H ; 7,38 ppm : d (J=8,1) : 1H ; 7,67 ppm : s : 1H ; 7,85 ppm : d (J=8,2) : 1H ; 8,15 ppm : d (J=8,2) : 1H ; 8,45 ppm : m : 2H ; 12,11 ppm : s : 1H.

### Exemple 22 : composé 143

### 3-(3-Acétylphényl)-1,6-diméthyl-1,9-dihydro-2H-pyrido[2,3-b]indol-2-one.

On dissout 400 mg du composé 60 dans 20 ml de THF anhydre. On refroidit le milieu réactionnel à 0°C, puis on ajoute 9,1 ml de CH₃Li 1,4M dans THF par portions. On laisse revenir à température ambiante, puis on agite 2 heures. On verse le milieu réactionnel sur HCl 1N, puis on lave à l'AcOEt. On basifie la phase aqueuse avec NaOH 5N, puis on extrait le produit avec de l'AcOEt. On sèche sur MgSO₄. On filtre et on évapore à sec. On purifie sur colonne de silice éluée avec un mélange AcOEt/éther de pétrole (v/v ; 80/20), puis avec de l'AcOEt pur. On obtient 40 mg du composé attendu, F = 258-260°C.

RMN DMSO (300 MHz) : 2,43 ppm : s : 3H ; 2,63 ppm : s : 3H ; 3,70 ppm : s : 3H ; 7,03-8,43 ppm : m : 8H ; 11,92 ppm : s : 1H.

### Exemple 23 : composé 127

### 1,6-Diméthyl-3-(3-(pyridin-2-ylamino)phényl)-1,9-dihydro-2H-pyrido[2,3-b] indol-2-one.

On dissout 500 mg du composé de l'Exemple 18 étape A, 180 mg de 2-aminopyridine, 315 mg de NaOtBu, 50 mg de Pd₂(dba)₃ et 95 mg de Xantphos dans 7 ml de dioxane (dans un tube scellé). On dégaze le milieu réactionnel pendant 30 minutes, puis on chauffe à 100°C pendant une nuit. On revient à température ambiante. Après purification sur colonne de silice éluée à l'acétate d'éthyle, on obtient 380 mg de poudre. F = 250-252°C.

RMN DMSO (300 MHz) : 2,42 ppm : s : 3H ; 3,70 ppm : s : 3H ; 6,71-8,27 ppm : m: 12H ; 9 ppm : s : 1H ; 11,88 ppm : s : 1H.

### Exemple 24 : composé 94

### (4-(1,6,9-Triméthyl-2-oxo-2,9-dihydro-1H-pyrido[2,3-b] indol-3-yl)phénoxy) acétonitrile.

On dissout 0,1 g du composé de 66 (exemple 9) dans 10 ml de DMF. On ajoute 90 ml de K₂CO₃ puis 0,12 ml de bromoacétonitrile. On chauffe à 90°C pendant 48 heures. On verse le milieu réactionnel sur une solution de NH₄Cl saturée, on alcalinise à la soude, puis on extrait à l'AcOEt. On purifie par chromatographie sur colonne de silice éluée avec un mélange AcOEt/éther de pétrole (v/v ; 75/25). On obtient 20 mg de poudre. F = 195-196°C

RMN DMSO (300 MHz) : 2,73 ppm : s : 3H ; 4,27 ppm : s : 6H ; 5,02 ppm : s : 2H ; 7,09-8,26 ppm : m : 8H.

### Exemple 25 : composé 133

### 1,6-Diméthyl-3-(3-(morpholin-4-ylcarbonyl)phényl)-1,9-dihydro-2H-pyrido[2,3-b]indol-2-one.

### A) Acide (3-(1,6-diméthyl-2-oxo-2,9-dihydro-1H-pyrido[2,3-b] indol-3-yl) benzoïque.

On dissout 200 mg du composé de l'exemple 18 dans 10 ml de MeOH. On ajoute 2 ml d'eau puis 97 mg de LiOH, H₂O. On chauffe à 80°C pendant 20 heures. On laisse revenir à température ambiante. On évapore le MeOH, on reprend dans l'eau, puis on lave la phase aqueuse avec de l'AcOEt. On basifie la phase aqueuse avec NaOH 5N, puis on extrait à l'AcOEt. On sèche la phase organique sur MgSO₄, on filtre et on évapore à sec. On triture le précipité obtenu avec un mélange éther de pétrole/AcOEt (2 %). On filtre et on sèche le précipité. On obtient 40 mg d'une poudre.

RMN DMSO (300 MHz) : 2,42 ppm : s : 3H ; 3,70 ppm : s : 3H ; 7,04-8,42 ppm : m : 8H ; 11,93 ppm : s : 1H ; 12,87 ppm : m : 1H.

### B) 1,6-Diméthyl-3-(3-(morpholin-4-ylcarbonyl)phényl)-1,9-dihydro-2H-pyrido[2,3-b]indol-2-one.

On dissout 200 mg du composé de l'étape précédente dans 20 ml de CH₂Cl₂. On ajoute 0,06 ml de morpholine, 340 mg de BOP et 10 ml de DMF. On agite le milieu réactionnel à température ambiante pendant 2 heures environ. On verse le milieu réactionnel sur NH₄Cl saturé, et on extrait à l'AcOEt. On fait un lavage acido-basique, puis on lave le précipité obtenu avec un mélange iPrOH/éther de pétrole (v/v ; 50/50). On obtient 90 mg de poudre. F = 296-298°C

RMN DMSO (300 MHz) : 2,42 ppm : s : 3H ; 3,38-3,62 ppm : s : 8H ; 3,69 ppm : s : 3H ; 7,03-8,42 ppm : m : 8H ; 11,92 ppm : s : 1H.

### Exemple 26 : composé 123

### 6-Cyano-3-(3,5-diméthylphényl)-1-méthyl-1,9-dihydro-2H-pyrido[2,3-b]indol-2-one.

### A) 6-Bromo-3-(3,5-diméthylphényl)-1-méthyl-1,9-dihydro-1H-pyrido[2,3-b] indol-2-one.

Ce composé est préparé par les méthodes habituelles.

RMN DMSO (300 MHz) : 2,31 ppm : s : 6H ; 3,69 ppm : s : 3H ; 6,91 ppm : s : 1H ; 7,28-7,44 ppm : m : 4H ; 8,15 ppm : d : 1H ; 8,38 ppm : s : 1H ; 12,30 ppm : s : 1H.

### B) 6-Cyano-3-(3,5-diméthylphényl)-1-méthyl-1,9-dihydro-2H-pyrido[2,3-b]indol-2-one.

On dissout 2,0 g du composé de l'étape précédente dans 50 ml de N-méthylpyrrolidinone. On rajoute 0,9 g de CuCN puis on chauffe à 200°C pendant 24 heures. On verse le milieu réactionnel sur du CH₂Cl₂ puis on lave avec une solution de HCl 1N. On adsorbe le produit sur silice et on purifie par chromatographie sur colonne éluée avec un mélange AcOEt/éther de pétrole (v/v ; 50/50) puis AcOEt pur puis AcOEt/MeOH 2 %. On obtient 1,2 g de poudre.

RMN DMSO (300 MHz) : 2,31 ppm : s : 6H ; 3,70 ppm : s : 3H ; 6,93 ppm : s : 1H ; 7,33 ppm : s : 2H ; 7,58 ppm : m : 2H ; 8,42 ppm : m : 2H ; 12,55 ppm : s : 1H.

### Exemple 27 : composé 96

### 3-(2,4-Dichlorophényl)-1,6-diméthyl-2-oxo-1,2-dihydro-9H-pyrido[2,3-b]indol-9-yl)acétonitrile.

On dissout 600 mg du composé 46 dans 10 ml de DMF. On ajoute 42 mg de NaH 95 %. On agite 30 minutes à TA puis on ajoute 230 mg de bromoacétonitrile. On agite 18 heures à TA. On concentre à moitié et on précipite par addition d'eau. On obtient 200 mg du produit attendu, F = 270°C.

### Exemple 28 : composé 92

### Chlorhydrate de 9-(3-Aminopropyl)-3-(2,4-dichlorophényl)-1,6-diméthyl-1,6-diméthyl-1,9-dihydro-2H-pyrido[2,3-b]indol-2-one.

### A) 2-(3-(3-(2,4-Dichlorophényl)-1,6-diméthyl-2-oxo-1,2-dihydro-9H-pyrido[2,3-b] indol-9-yl)propyl)-1H-isoindole-1,3-(2H)dione.

On dissout 0,5 g du composé 46 dans 5 ml de DMF, on ajoute 107 mg de NaH 95 % et on agite 10 minutes à TA. On ajoute 1,25 g de 2-(3-bromopropyl)-1*H*-isoindole-1,3(2*H*)dione et on agite 18 heures à TA. On évapore, on reprend dans CH₂Cl₂, on lave par NaCl puis H₂O. On chromatographie sur silice en éluant par CHCl₃/MeOH (v/v ; 98/2). On obtient 160 mg du produit attendu, F = 249°C.

### B) Chlorhydrate de 9-(3-Aminopropyl)-3-(2,4-dichlorophényl)-1,6-diméthyl-1,6-diméthyl-1,9-dihydro-2H-pyrido[2,3-b]indol-2-one.

On mélange 180 mg du composé de l'étape précédente, 3,2 ml de THF, 5 ml d'EtOH et 33 µl d'hydrate d'hydrazine. On chauffe à reflux 18 heures ; on essore, on évapore la phase HCl. On sèche. On obtient 16 mg du produit attendu, F = 198°C.

### Exemple 29 : composé 91

### 3-(2,4-Dichlorophényl)-9-(2-hydroxyéthyl)-1,6-diméthyl-1,9-dihydro-2H-pyrido[2,3-b]indol-2-one.

### A) 3-(2,4-Dichlorophényl)-1,6-diméthyl-9-tétrahydro-2H-pyran-2-yloxy)éthyl)-1,9-dihydro-2H- pyrido[2,3-b]indol-2-one.

On dissout 695 mg du composé 46 dans 7 ml de DMF. On ajoute 99 mg de NaH 95 %. On agite 15 minutes à TA. On ajoute 813 mg de 2-(2-bromoéthoxy)tétrahydro-2*H*-pyrane et on agite 18 heures à TA. On évapore à sec, on reprend par CH₂Cl₂, on lave par NaCl, puis H₂O. On sèche, on évapore. On chromatographie par CHCl₃/MeOH (v/v ; 98/2). On obtient 390 mg du produit attendu, F =161°C.

### B) 3-(2,4-Dichlorophényl)-9-(2-hydroxyéthyl)-1,6-diméthyl-1,9-dihydro-2H-pyrido[2,3-b]indol-2-one.

On mélange 295 mg du produit de l'étape précédente, 1,21 mg d'acide p-toluène sulfonique et 10 ml d'EtOH 95. On chauffe à reflux 8 heures, on évapore à sec. On lave le solide par NaHCO₃, puis H₂O, on sèche. On obtient 198 mg du produit attendu, F = 240°C.

### Exemple 30 : composé 102

### 3-(2,4-Diméthylphényl)-1,6,9-triméthyl-1,9-dihydro-2H-pyrido[2,3-b]indol-2-one.

On dissout 400 mg de 3-(2,4-diméthylphényl)-1,6-diméthyl-1,9-dihydro-2*H-*pyrido[2,3-b]indol-2-one (composé 101) dans 10 ml de DMF. On ajoute 31 mg de NaH 95 %, on agite 30 minutes à TA. On ajoute 380 µl d'iodure de méthyle et on agite 18 heures à TA. On évapore à sec. On reprend par CH₂Cl₂, on lave par NaCl puis H₂O, on sèche, on évapore. On reprend par Et₂O, on essore, on sèche. On obtient 300 mg du produit attendu, F = 150°C.

### Exemple 31 : composé 57

### 3-(2-chloro-4-méthoxyphényl)-1,6-diméthyl-1,9-dihydro-2H-pyrido[2,3-b]indol-2-one.

On prépare un mélange de 10 ml de DMF anhydre et 0,6 ml de méthanol anhydre auquel on ajoute 0,5 g de NaH à 60 %. A la fin du dégagement gazeux on ajoute 0,43 g du composé 59 puis on chauffe à 80°C pendant 24 heures. On verse le milieu réactionnel sur une solution saturée de NH₄Cl puis on filtre le précipité formé et on le lave par un mélange isopropanol/éther de pétrole (v/v ; 50/50). On obtient 0,35 g du composé attendu.

RMN DMSO (300 MHz) : 2,46 ppm : s : 3H ; 3,72 ppm : s : 3H ; 3,87 ppm : s : 3H ; 6,98-7,15 ppm : m : 3H ; 7,3-7,6 ppm : m : 2H ; 7,68 ppm : s : 1H ; 8,15 ppm : s : 1H ; 11,95 ppm : s : 1H.

Le tableau qui suit illustre les structures chimiques et les propriétés physiques de quelques composés selon l'invention. Dans ce tableau Me représente méthyle, Et représente éthyle, tBu représente *tert*-butyle, Bn représente benzyle.

**TABLEAU 4**

| | | | | | |
|---|---|---|---|---|---|
| 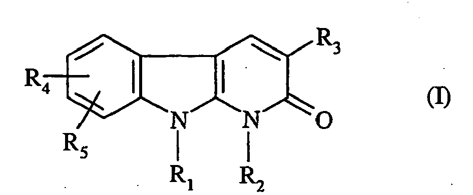 | | | | | |

| Composés | R₁ | R₂ | R₃ | R₄, R₅ | Caractérisation F°C ou RMN |
|---|---|---|---|---|---|
| 1 | Me | Me | 3,5-diMe-phényl | 6-Me | 210°C |
| 2 | Me | Me | 2,4-diCl-phényl | 6-Me | 170°C |
| 3 | Me | Me | 3,4-diCl-phényl | 6-Me | 230°C |
| 4 | Me | Me | 3-CF₃-phényl | 6-Me | 186°C |
| 5 | Me | Me | 3,5-diCF₃-phényl | 6-Me | 248°C |
| 6 | Me | Me | 3,5-diF-phényl | 6-Me | 240°C |
| 7 | Me | Me | 3,4,5-triOMe-phényl | 6-OMe | 224°C dec |
| 8 | Me | Me | 1,3-benzodioxol-5-yl | 6-OMe | 197°C |
| 9 | Me | Me | 2,5-diOMe-phényl | 6-Me | 225°C |
| 10 | Me | Me | 3,5-diMe-phényl | 6-Cl | 200°C |
| 11 | Me | Me | 1,3-benzodioxol-5-yl | 7-OMe | 220°C dec |
| 12 | Me | Me | 3,4-diOMe-phényl | 7-OMe | 190°C |
| 13 | Me | Me | 3,4,5-triOMe-phényl | 8-OMe | 219°C |
| 14 | Me | Me | 3,4-diOMe-phényl | 6-Me | 190°C |
| 15 | Me | Me | 3,5-diOMe-phényl | 6-Me | 205°C |
| 16 | H | Me | 3,5-diMe-phényl | 6-Me | RMN |
| 17 | Me | Me | 3,5-diOMe-phényl | 6-Cl | 229 |
| 18 | Me | Me | 3,5-diF-phényl | H | 232°C |
| 19 | Me | Me | 2,4-diF-phényl | 6-Me | 167°C |
| 20 | Me | Me | 3,5-diF-phényl | 6-OMe | 189°C |
| 21 | Me | Me | 2,4-diCl-phényl | 6-OMe | 130°C |
| 22 | H | Me | 3,5-diF-phényl | 6-Me | 189°C dec |
| 23 | Me | Me | 3,5-diMe-phényl | 6-OMe | 194°C |
| 24 | Me | Me | 3-F-5-CF₃-phényl | 6-Me | 190°C |
| 25 | Me | Me | 3,5-diF-phényl | 5-OMe | 238°C |
| 26 | Me | Me | 1,3-benzodioxol-5-yl | 8-OMe | 197°C |
| 27 | Me | Me | 3,5-diF-phényl | 7-OH | 195°C |
| 28 | Me | Me | 2,4-diCl-phényl | H | 241 °C |
| 29 | Me | Me | 2,4-diCl-phényl | 7-OMe | RMN |
| 30 | Me | Me | 2,4-diCl-phényl | 5-OMe | 228°C |
| 31 | Me | Me | 2,4-diCl-phényl | 7-Cl | RMN |
| 32 | Me | Me | 2,4-diCl-phényl | 5,7-diMe | 240°C |
| 33 | Me | Me | 2,4-diCl-phényl | 6-Cl | 258°C |
| 34 | Me | Me | 3,5-diF-phényl | 6-Cl | 295°C |
| 35 | H | Me | 3,5-diF-phényl | H | 262°C |
| 36 | H | Me | 2,4-diCl-phényl | H | RMN |
| 37 | Me | Me | 3,5-diMe-phényl | 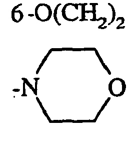 | RMN |
| 38 | Me | Me | 3,5-diCl-phényl | 6-Me | 268°C |
| 39 | Me | Me | 2,3-diF-phényl | 6-Me | 170°C |
| 40 | Me | Me | 2,3-diF-phényl | 6-OMe | 179°C |
| 41 | H | Me | 3-CF₃, 5-F-phényl | 6-Me | 270°C |
| 42 | Me | Me | 3-OH-phényl | 6-Me | RMN |
| 43 | Me | Me | 2-Cl, 4F-phényl | 6-Me | 202-203°C |
| 44 | Me | Me | 4-SO₂Me-phényl | 6-Me | 245-246°C |
| 45 | Me | Me | 2-Cl, 4-OMe-phényl | 6-Me | 196-197°C |
| 46 | H | Me | 2,4-diCl-phényl | 6-Me | RMN |
| 47 | Me | Me | 4-OH, 3-tBu-phényl | 6-Me | RMN |
| 48 | Me | Me | 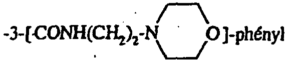 | 6-Me | 194°C |
| 49 | Me | Me | 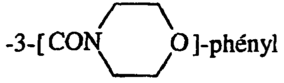 | 6-Me | 252°C |
| 50 | Me | Me | 3-CH₂NH₂-phényl | 6-Me | RMN |
| 51 | Me | Me | 3-CONH₂-phényl | 6-Me | 269°C |
| 52 | Me | Me | 3-CN-phényl | 6-Me | 241-242°C |
| 53 | Me | Me | 4-(CH₂NH₂)-phényl | 6-Me | 213-214°C |
| 54 | H | Me | 4-SO₂Me-phényl | 6-Me | 278°C |
| 55 | H | Me | 3,4,5-triOMe-phényl | 6-Me | RMN |
| 56 | H | Me | 4-(CH₂NH₂)-phényl | 6-Me | 258°C |
| 57 | H | Me | 2-Cl, 4-OMe-phényl | 6-Me | RMN |
| 58 | H | Me | 4-CN-phényl | 6-Me | RMN |
| 59 | H | Me | 2-Cl, 4-F-phényl | 6-Me | RMN |
| 60 | H | Me | 3-CN-phényl | 6-Me | 295°C |
| 61 | H | Me | 3-(CH₂NH₂)-phényl | 6-Me | RMN |
| 62 | H | Me | 4-COOMe-phényl | 6-Me | RMN |
| 63 | H | Me | 2-Cl, 4-OH-phényl | 6-Me | RMN |
| 64 | H | Me | 3-NH₂-phényl | 6-Me | RMN |
| 65 | H | Me | 4-COOH-phényl | 6-Me | RMN |
| 66 | Me | Me | 4-OH-phényl | 6-Me | 280°C |
| 67 | Me | Me | 4-NMe₂-phényl | 6-Me | 118°C |
| 68 | H | Me | 4-OH-phényl | 6-Me | 186°C |
| 69 | Me | Me | 2,6-diCl-phényl | 6-Me | 255°C |
| 70 | Me | Me | 4-[CONH(CH₂)₂ N(CH₃)₂]-phényl | 6-Me | 105°C |
| 71 | Me | Me | 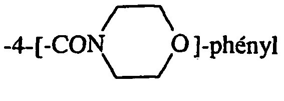 | 6-Me | 174°C |
| 72 | Me | Me | 3-COOMe-phényl | 6-Me | 172-173°C |
| 73 | Me | Me | 2-Cl, 4-OH-phényl | 6-Me | RMN |
| 74 | Me | Me | 2,4-diCl-phényl | 6-CO₂Me | 270°C |
| 75 | H | Me | 4-SMe-phényl | 6-Me | 280°C |
| 76 | H | Me | 4-N(CH₃)₂-phényl | 6-Me | 280°C |
| 77 | H | Me | 3,4-diOMe-phényl | 6-Me | RMN |
| 78 | CH₂-COO- -tBu | Me | 2,4-diCl-phényl | 6-Me | 113°C |
| 79 | Me | Me | 2,4-diCl-phényl | 6-CN | 290°C |
| 80 | H | Me | 3-CH₂NH(CH₃)-phényl | 6-Me | RMN |
| 81 | H | Me | 3-CH₂OH-phényl | 6-Me | RMN |
| 82 | H | Me | (3-Br, 4-OMe)-phényl | 6-Me | RMN |
| 83 | H | Me | 3-(CH=NOH)-phényl | 6-Me | 290°C |
| 84 | CH₂- -COO- -Me | Me | 2,4-diCl-phényl | 6-Me | 249°C |
| 85 | CH₂- -CONH -Me | Me | 2,4-diCl-phényl | 6-Me | RMN |
| 86 | Me | Me | 2,4-diCl-phényl | 6-phényl | 110°C |
| 87 | H | Me | 2-Br, 4,5-diOMe-phényl | 6-Me | RMN |
| 88 | Me | Me | 3-CN-phényl | 6-CN | > 280°C RMN |
| 89 | Me | Me | 2,Cl, 3,4-diOMe-phényl | Me | 250°C |
| 90 | 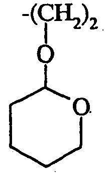 | Me | 2,4-diCl-phényl | 6-Me | 161°C |
| 91 | CH₂CH₂-OH | Me | 2,4-diCl-phényl | 6-Me | 240°C |
| 92 | (CH₂)₃-NH₂ | Me | 2,4-diCl-phényl | 6-Me | 198°C Chlorhydrate |
| 93 | Me | Me | 2-Br, 4,5-diOMe-phényl | 6-Me | 236°C |
| 94 | Me | Me | 4-(-OCH₂CN)-phényl | 6-Me | 192°C RMN |
| 95 | Me | Me | 4-(-OCH₂CH₂NH₂)-phényl | 6-Me | 170°C |
| 96 | CH₂CN | Me | 2,4-diCl-phényl | 6-Me | 277°C |
| 97 | CH₂CH₂-NH₂ | Me | 2,4-diCl-phényl | 6-Me | RMN |
| 98 | H | Me | 2-Cl, 4,5-OMe-phényl | 6-Me | RMN |
| 99 | Me | Me | 2-Cl, 4,5-diOMe-phényl | 6-Me | 245°C RMN |
| 100 | H | Me | 4-OMe, 3-CN-phényl | 6-Me | RMN |
| 101 | H | Me | 2,4-diMe-phényl | 6-Me | RMN |
| 102 | Me | Me | 2,4-diMe-phényl | 6-Me | 150°C |
| 103 | H | Me | 3,4-diMe-phényl | 6-Me | RMN |
| 104 | Me | Me | 3,4-diMe-phényl | 6-Me | 211 °C |
| 105 | Me | Me | 4-N₃-phényl | 6-Me | 184°C |
| 106 | Me | Me | 3,4-diOMe-phényl | 6-OMe | 160°C |
| | Me | Me | 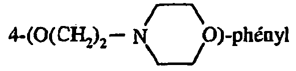 | 6-Me | 180°C Chlorhydrate |
| 108 | Me | Me | 2,3-diF-phényl | 6-OMe | 179°C |
| 109 | Me | Me | 3-tBu, 4-OMe-phényl | 6-Me | 231-232°C |
| 110 | H | Me | 3-COOMe-phényl | 6-Me | 278-280°C |
| 111 | H | Me | 4-NH₂-phényl | 6-Me | RMN |
| 112 | H | Me | 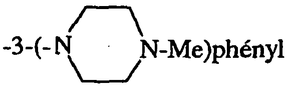 | 6-Me | 226°C |
| 113 | H | Me | 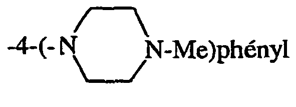 | 6-Me | RMN |
| 114 | H | Me | 2,4-diCl-phényl | 6-Br | 386°C dec RMN |
| 115 | H | Me | 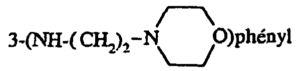 | 6-Me | 267-269°C |
| 116 | H | Me | 6-Cl-1,3-benzodioxol-5-yl | 6-Me | RMN |
| 117 | H | Me | 3,5-diOMe-phényl | 6-Me | 299°C RMN |
| 118 | H | Me | 3-(-NH-CH₂CH₂-NMe₂)-phényl | 6-Me | 237-239°C |
| 119 | H | Me | 4-N₃-phényl | 6-Me | RMN |
| 120 | H | Me | 2,4-diOMe-phényl | 6-Me | 308-310°C |
| 121 | H | Me | 3-(NH-C₆H₅)-phényl | 6-Me | 188-190°C |
| 122 | H | Me | 3,5-diMe-phényl | 6-CH₂NH₂ | RMN |
| 123 | H | Me | 3,5-diMe-phényl | 6-CN | RMN |
| 124 | Me | Me | 4-NH₂-phényl | 6-Me | 224°C RMN |
| 125 | H | Me | 3,5-diMe-phényl | 6-Br | 320°C dec RMN |
| 126 | H | Me | 3-CF₃-phényl | 6-Me | 246°C RMN |
| 127 | H | Me | 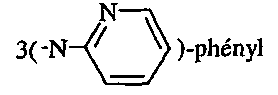 | 6-Me | 250°C |
| 128 | H | Me | 2,4-diCl-phényl | 6-OMe | 318°C RMN |
| 129 | H | Me | 3-(-OCH₂C₆H₅)-phényl | 6-Me | 221 °C RMN |
| 130 | H | Me | 3-OH-phényl | 6-Me | 310°C dec RMN |
| 131 | H | Me | 3,5-diCl-phényl | 6-Me | 300-302°C RMN |
| 132 | H | Me | 3,5-diMe-phényl | 6-OMe | 317°C dec |
| 133 | H | Me | 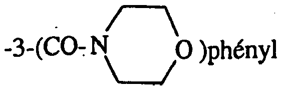 | 6-Me | 296-298°C |
| 134 | H | Me | 4-CONH₂-phényl | 6-Me | RMN |
| 135 | Me | Me | 3-NH₂-phényl | 6-Me | 133-135°C |
| 136 | H | Me | 3,5-diMe-phényl | 6-CH₂NMe₂ | 238-240°C |
| 137 | H | Me | 3-(-OCH₂COOMe)-phényl | 6-Me | 208-209°C |
| 138 | Me | Me | 3-(-COMe)-phényl | 6-Me | 80°C dec RMN |
| 139 | H | Me | 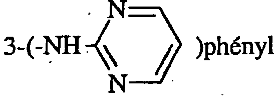 | 6-Me | 190°C dec |
| 140 | H | Me | 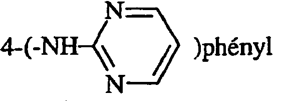 | 6-Me | 316°C dec RMN |
| 141 | CH₂CN | Me | 3-(OCH₂CN)-phényl | 6-Me | 225-227°C RMN |
| 142 | (CH₂)₂N-(CH₃)₂ | Me | 3-OH-phényl | 6-Me | 216-218°C RMN |
| 143 | H | Me. | 3-(-COMe)phenyl | 6-Me | 258-260°C |
| 144 | Me | Me | 3-Br, 4-OMe-phényl | 6-Me | 245-247°C |
| 145 | H | Me | 3-N₃-phényl | 6-Me | RMN |
| 146 | Me | Me | 3-N₃-phényl | 6-Me | 188°C |
| 147 | H | Me | 2,4-diCN-phényl | 6-Me | RMN |
| 148 | H | Me | 2-Cl, 4-Br-phényl | 6-Me | RMN |
| 149 | Me | Me | 2-Cl, 4-Br-phényl | 6-Me | 251°C |
| 150 | R₁+R₂=-(CH₂)₃ | | 2,4-diCl-phényl | 6-Me | 197-198°C RMN |
| 151 | Me | Me | 2,4-diCN-phényl | 6-Me | 292°C |
| 152 | H | Me | 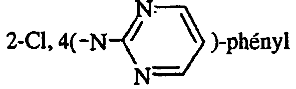 | 6-Me | 291 °C |
| 153 | Me | Me | 2,4-diCl-phényl | 6-Br | 228°C |
| 154 | 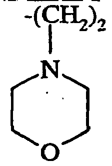 | Me | 2,4-diCl-phényl | 6-Br | 179-180°C dec |
| 155 | CH₂CN | Me | 2,4-diCl-phényl | 6-COOEt | RMN |
| 156 | H | Me | 2,4-diCl-phényl | 6-COOEt | RMN |
| 157 | 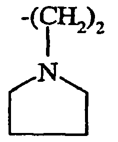 | Me | 2,4-diCl-phényl | 6-Br | 210°C |
| 158 | H | Me | 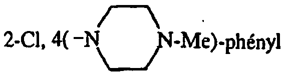 | 6-Me | 250°C dec |
| 159 | H | Me | 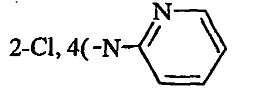 | 6-Me | RMN |
| 160 | H | Me | 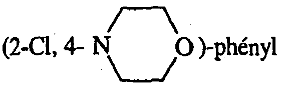 | 6-Me | RMN |
| 161 | Me | Me | 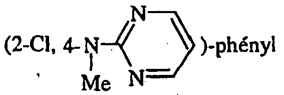 | 6-Me | 262-264°C |
| 162 | H | Me | 2,4-diCl-phényl | 6-COOMe | 322-324°C |
| 163 | H | Me | 2,4-diCl-phényl | 6-SO₂Me | 338-340°C |
| 164 | Me | Me | 2,4-diCl-phényl | 6-SO₂Me | 156-160°C |
| 165 | H | Me | 2,4-diOMe-phényl | 6-Br | 207-208°C |
| 166 | H | Me | 3,5-diCl-phényl | 6-COOMe | RMN |
| 167 | H | Me | 2-Cl, 4-Br-phényl | 6-COOMe | 321-323°C |
| 168 | H | Me | 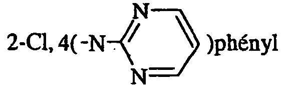 | 6-COOMe | 297-298°C |
| 169 | H | Me | 2,4-diOMe-phényl | 6-CN | 239-242°C |

Composé 16 : : RMN DMSO (300 MHz) : 2,3 ppm : s : 6H ; 2,4 ppm : s : 3H ; 3,67 ppm : s : 3H ; 6,9 ppm : s : 1H ; 7 ppm : d : 1H ; 7,3 ppm : m : 3H ; 7,7 ppm : s : 1H ; 8,26 ppm : s : 1H ; 11,9 ppm : s : 1H.
Composé 29 : : RMN CDCl₃ (300 MHz) : 3,93 ppm : s : 3H ; 4,09 ppm : s : 6H ; 6,89 ppm : d : 1H ; 6,92 ppm : dd : 1H ; 7,3 ppm : dd : 1H ; 7,38 ppm : d : 1H ; 7,51 ppm : d : 1H ; 7,62 ppm : d : 1H ; 7,94 ppm : s : 1H.
Composé 31 : RMN DMSO (300 MHz) : 4 ppm : s : 3H ; 4,15 ppm : s : 3H ; 7,23 ppm : dd : 1H ; 7,42 ppm : d : 1H ; 7,47 ppm : dd : 1H ; 7,68 ppm : d : 1H ; 7,78 ppm : d : 1H ; 7,88 ppm : d : 1H ; 8,23 ppm : s : 1H.
Composé 36 : RMN CDCl₃ (300 MHz) : 3,83 ppm : s : 3H ; 7,29-7,49 ppm : m : 6H ; 7,76 ppm : m : 1H ; 8,03 ppm : s : 1H ; 8,59 ppm : s : 1H.
Composé 37 : RMN DMSO (300 MHz) : 2,41 ppm : s : 6H ; 2,63-2,68 ppm : m : 4H ; 2,89 ppm : t (J=5,8) : 2H ; 3,77-3,82 ppm : m : 4H ; 4,08 ppm : m : 6H ; 4,24 ppm : t (J=5,8) : 2H ;6,95-8,05 ppm : m : 7H.
Composé 42 : RMN DMSO (200 MHz) : 2,41 ppm : s : 3H ; 3,7-4,5 ppm : se : 1H ; 3,97 ppm : s : 3H ; 4,07 ppm : s : 3H ; 6,65 ppm : d : 1H ; 7-7,3 ppm : m : 4H ; 7,4 ppm : d : 1H ; 7,7 ppm : s : 1H ; 8,25 ppm : s : 1H.
Composé 46 : RMN DMSO (300 MHz) : 2,5 ppm : s : 3H ; 3,7 ppm : s : 3H ; 7,1 ppm : d : 1H ; 7,3-7,5 ppm : m : 3H ; 7,7 ppm : d : 2H ; 8,1 ppm : s : 1H ; 11,9 ppm : s : 1H.
Composé 47 : RMN CDCl₃ (300 MHz) : 1,45 ppm : s : 9H ; 2,5 ppm : s : 3H ; 4,1 ppm : s : 6H ; 6,7 ppm : d : 1H ; 7,2 ppm : d : 1H ; 7,3 ppm : d : 1H ; 7,5 ppm : d : 1H ; 7,6 ppm : d : 1H ; 8 ppm : s : 1H.
Composé 50 : RMN CDCl₃ (300 MHz) : 1,7 ppm : m : 2H ; 2,5 ppm : s : 3H ; 4,1 ppm : s : 6H ; 7,1 ppm : d : 1H ; 7,3 ppm : m : 2H ; 7,4 ppm : t : 1H ; 7,6 ppm : m : 2H ; 7,7 ppm : s : 1H ; 8,1 ppm : s : 1H.
Composé 55 : RMN DMSO (300 MHz) : 2,43 ppm : s : 3H ; 3,69 ppm : s : 6H ; 3,83 ppm : s : 6H ; 7,03 ppm : d : 1H ; 7,11 ppm : s : 2H ; 7,33 ppm : d : 1H ; 7,71 ppm : s : 1H ; 8,37 ppm : s : 1H ; 11,86 ppm : s : 1H.
Composé 58 : RMN DMSO (300 MHz) : 2,43 ppm : s : 3H ; 3,70 ppm : s : 3H ; 7,05 : d : 1H ; 7,3 ppm : d : 1H ; 7,73 ppm : s : 1H ; 7,8 ppm : d : 2H ; 8,03 ppm : d : 2H ; 8,54 ppm : s : 1H ; 12 ppm : s : 1H.
Composé 59 : RMN DMSO (300 MHz) : 2,40 ppm : s : 3H ; 3,67 ppm : s : 3H ; 7,02-7,06 : m : 1H ; 7,24-7,28 ppm : m : 1H ; 7,34 ppm : d : 1H ; 7,42-7,47 ppm : m : 2H ; 7,63 ppm : s : 1H ; 8,1 ppm : s : 1H ; 11,95 ppm : se : 1H.
Composé 61 : RMN DMSO (300 MHz) : 2,40 ppm : s : 3H ; 3,25 ppm : s : 6H ; 7,02: d : 1H ; 7,23-7,36 ppm : m : 3H ; 7,61-7,68 ppm : m [soit 7,05 ppm : d (J=8,1) : 1H ; 7,35 ppm : d (J=8,1) : 1H ; 7,73 ppm : s : 1H ; 7,78 ppm : m : 4H] : 3H ; 8,3 ppm : s : 1H.
Composé 62 : RMN DMSO (300 MHz) : 2,43 ppm : s : 3H ; 3,70 ppm : s : 3H ; 3,88 ppm : s : 3H ; 7,05-8,50 ppm : m : 8H ; 11,97 ppm : s : 1H.
Composé 63 : RMN DMSO (300 MHz) : 2,41 ppm : s : 3H ; 3,66 ppm : s : 3H ; 6,75-9,80 ppm : m : 8H ; 11,87 ppm : s : 1H.
Composé 64 : RMN DMSO (300 MHz) : 2,42 ppm : s : 3H ; 3,67 ppm : s : 3H ; 4,88 ppm : m : 2H ; 6,47-8,16 ppm : m : 8H [soit 6,49 ppm : d (J=7,5) : 1H ; 6,84 ppm : d (J=7,5) : 1H ; 6,92-7,15 ppm : m : 3H ; 7,34 ppm : d (J=8,1) : 1H ; 7,65 ppm : m ; 1H ; 8,18 ppm : m : 1H] ; 11,82 ppm : m : 1H.
Composé 65 : RMN DMSO (300 MHz) : 2,43 ppm : s : 3H ; 3,707 ppm : s : 3H ; 7,04-8,47 ppm : m : 8H ; 11,95 ppm : s : 1H.
Composé 73 : RMN DMSO (300 MHz) : 2,3 ppm : s : 3H ; 4 ppm : s : 3H ; 4,1 ppm : s : 3H ; 6,8 ppm : d : 1H ; 6,9 ppm : s : 1H ; 7,2 ppm : d : 1H ; 7,3 ppm : d : 1H ; 7,5 ppm : d : 1H ; 7,7 ppm : s : 1H ; 8,1 ppm : s : 1H ; 9,9 ppm : se : 1H.
Composé 77 : RMN DMSO (200 MHz) : 2,4 ppm : s : 3H ; 3,7 ppm : s : 3H ; 3,8 ppm : s : 6H ; 6,9 ppm : d : 1H ; 7 ppm : d : 1H ; 7,2-7,3 ppm : m : 3H ; 7,4 ppm : s : 1H ; 8,3 ppm : s : 1H.
Composé 80 : RMN DMSO + TFA (200 MHz) : 2,4 ppm : s : 3H ; 2,8 ppm : s : 3H ; 3,8 ppm : s : 3H ; 4,3-4,5 ppm : dd : 2H ; 7,1 ppm : d : 1H ; 7,3-7,6 ppm : m : 3H ; 7,7 ppm : s : 1H ; 7,9 ppm : d : 1H ; 8 ppm : s : 1H ; 8,4 ppm : s : 1H.
Composé 81 : RMN DMSO (200 MHz) : 2,3 ppm : s : 3H ; 3,6 ppm : s : 3H ; 4,4 ppm : d : 2H ; 5,1 ppm : m : 1H ; 7 ppm : d : 1H ; 7,15-7,3 ppm : m : 3H ; 7,58-7,7 ppm : m : 3H ; 8,2 ppm : s : 1H ; 11,8-12 ppm : se : 1H.
Composé 82 : RMN DMSO (200 MHz) : 2,4 ppm : s : 3H ; 3,6 ppm : s : 3H ; 3,8 ppm : s : 3H ; 6,95 ppm : d : 1H ; 7,05 ppm : d : 1H ; 7,15 ppm : d : 1H ; 7,6-7,8 ppm : m : 2H ; 8 ppm : s : 1H ; 8,3 ppm : s : 1H ; 11,8 ppm : s : 1H.
Composé 85 : RMN DMSO (200 MHz) : 2,3 ppm : s : 3H ; 2,6 ppm : d : 3H ; 3,8 ppm : s : 3H ; 5,2 ppm : s : 2H ; 7 ppm : d : 1H ; 7,3 ppm : d : 1H ; 7,4 ppm : m : 2H ; 7,6 ppm : s : 2H ; 8,1 ppm : s : 1H ; 8,4 ppm : d : 1H.
Composé 87 : RMN DMSO/TFA (200 MHz) : 2,4 ppm : s : 3H ; 3,6 ppm : s : 3H ; 3,95 ppm : s : 3H ; 7 ppm : d : 1H ; 7,15-7,35 ppm : m : 2H ; 7,65 ppm : s : 1H ; 8,05 ppm : s : 1H ; 8,1 ppm : s : 1H ; 8,45 ppm : s : 1H.
Composé 88 : RMN DMSO/TFA (200 MHz) : 4,05 ppm : s : 3H ; 4,25 ppm : s : 3H ; 7,6-7,9 ppm : m : 4H ; 8,15 ppm : d : 1H ; 8,25 ppm : s : 1H ; 8,5 ppm : s : 1H ; 8,7 ppm : s : 1H.
Composé 94 : RMN DMSO (300 MHz) : 2,73 ppm : s : 3H ; 4,27 ppm : s : 6H ; 5,02 ppm : s : 2H ; 7,09-8,26 ppm : m : 8H.
Composé 97 : RMN DMSO/TFA (200 MHz) : 2,4 ppm : s : 3H ; 2,95 ppm : t : 2H ; 4 ppm : s : 3H ; 4,6 ppm : t : 2H ; 7,2 ppm : d : 1H ; 7,4-7,7 ppm : m : 3H ; 7,75 ppm : s : 2H ; 8,2 ppm : s : 1H.
Composé 98 : RMN DMSO/TFA (200 MHz) : 2,4 ppm : s : 3H ; 3,6 ppm : s : 3H ; 3,7 ppm : s : 3H ; 3,8 ppm : s : 3H ; 6,95 ppm : s : 1H ; 7-7,1 ppm : m : 2H ; 7,35 ppm : d : 1H ; 7,6 ppm : s : 1H ; 8,05 ppm : s : 1H.
Composé 99 : RMN DMSO/TFA (200MHz) : 2,3 ppm : s : 3H ; 3,7 ppm : s : 3H ; 3,75 ppm : s : 3H ; 3,9 ppm : s : 3H ; 4,05 ppm : s : 3H ; 6,85 ppm : s : 1H ; 7 ppm : s : 1H ; 7,05 ppm : s : 1H ; 7,4 ppm : d : 1H ; 7,6 ppm : s : 1H ; 8 ppm : s : 1H.
Composé 100 : RMN DMSO/TFA (200 MHz) : 2,4 ppm : s : 3H ; 3,65 ppm : s : 3H ; 3,7 ppm : s : 3H ; 3,8 ppm : s : 3H ; 6,95 ppm : s : 1H ; 7,05 ppm : d : 1H ; 7,2 ppm : s : 1H ; 7,35 ppm : d : 1H ; 7,65 ppm : s : 1H ; 8,05 ppm : s : 1H.
Composé 101 : RMN DMSO/TFA (200 MHz) : 2,2 ppm : s : 3H ; 2,3 ppm : s : 3H ; 2,4 ppm : s : 3H ; 3,75 ppm : s : 3H ; 7-7,2 ppm : m : 4H ; 7,4 ppm : d : 1H ; 7,7 ppm : s : 1H ; 8 ppm : s : 1H.
Composé 103 : RMN DMSO/TFA (200 MHz) : 2,3 ppm : s : 3H ; 2,45 ppm : s : 3H ; 2,5 ppm : s : 3H ; 3,7 ppm : s : 3H ; 7,1 ppm : d : 1H ; 7,2 ppm : d : 1H ; 7,4 ppm : d : 1H ; 7,5 ppm : d : 1H ; 7,6 ppm : s : 1H ; 7,75 ppm : s : 1H ; 8,35 ppm : s : 1H.
Composé 112 : RMN DMSO (300 MHz) : 2,41 ppm : s : 3H ; 2,46 ppm : s : 3H ; 2,69-2,72 ppm : m : 4H ; 3,19-3,28 ppm : m : 4H ; 3,76 ppm : s : 3H ; 6,93 ppm : dd : 1H ; 7,09 ppm : d : 1H ; 7,15 ppm : d : 1H ; 7,28 ppm : d : 1H ; 7,33 ppm : m : 2H ; 7,61 ppm : s: 1H ; 8,19 ppm : s: 1H.
Composé 114 : RMN DMSO (300 MHz) : 3,69 ppm : s : 3H ; 7,4 ppm : m : 4H ; 7,67 ppm : d : 1H ; 8,09 ppm : d : 1H ; 8,29 ppm : s : 1H ; 12,28 ppm : s : 1H.
Composé 116 : RMN DMSO (300 MHz) : 2,41 ppm : s : 3H ; 3,67 ppm : s : 3H ; 6,12 ppm : s : 2H ; 7,02 ppm : s : 1H ; 7,10 ppm : s : 1H ; 7,19 ppm : s : 1H ; 7,36 ppm : d : 1H ; 7,63 ppm : s : 1H ; 8,05 ppm : s : 1H ; 11,92 ppm : s : 1H.
Composé 117 : RMN DMSO (300 MHz) : 2,42 ppm : s : 3H ; 3,68 ppm : s : 3H ; 3,78 ppm : s : 6H ; 6,41-8,37 ppm : m : 7H ; 11,89 ppm : s : 1H.
Composé 119 : RMN DMSO (200 MHz) : 2,4 ppm : s : 3H ; 3,7 ppm : s : 3H ; 7-7,2 ppm : m : 3H ; 7,4 ppm : d : 1H ; 7,7 ppm : s : 1H ; 7,8 ppm : d : 2H ; 8,4 ppm : s : 1H.
Composé 122 : RMN DMSO (300 MHz) : 2,50 ppm : s : 6H ; 3,79 ppm : s : 3H ; 4,03 ppm : m : 2H ; 6,92-8,28 ppm : m : 7H ; 12,46 ppm : s : 1H.
Composé 124 : RMN DMSO (300 MHz) : 2,43 ppm : s : 3H ; 3,98 ppm : s : 3H ; 4,08 ppm : s : 3H ; 7,06-7,16 ppm : m : 8H.
Composé 125 : RMN DMSO (300 MHz) : 2,31 ppm : s : 6H ; 3,69 ppm : s : 3H ; 6,91 ppm : s : 1H ; 7,28-7,44 ppm : m : 4H ; 8,15 ppm : d : 1H ; 8,38 ppm : s : 1H ; 12,30 ppm : s : 1H.
Composé 126 : RMN DMSO (300 MHz) : 2,43 ppm : s : 3H ; 3,70 ppm : s : 3H ; 7,05-8,88 ppm : m : 8H ; 11,97 ppm : s : 1H.
Composé 128 : RMN DMSO (300 MHz) : 3,61 ppm : s : 3H ; 3,78 ppm : s : 3H ; 6,83 ppm : dd : 1H ; 7,33-7,48 ppm : m : 4H ; 7,66 ppm : d : 1H ; 8,17 ppm : s: 1H ; 11,91 ppm : s : 1H.
Composé 129 : RMN DMSO (300 MHz) : 2,42 ppm : s : 3H ; 3,69 ppm : s : 3H ; 5,15 ppm : s : 2H ; 6,93-8,36 ppm : m : 13H ; 11,88 ppm : s : 1H.
Composé 130 : RMN DMSO (300 MHz) : 2,42 ppm : s : 3H ; 3,68 ppm : s : 3H ; 6,67 ppm : dd : 1H ; 7,02 ppm : dd : 1H ; 7,03-7,24 ppm : m : 3H ; 7,34 ppm : d : 1H ; 7,68 ppm : s : 1H ; 8,25 ppm : s : 1H ; 9,26 ppm : se : 1H ; 11,85 ppm : se : 1H.
Composé 131 : RMN DMSO (300 MHz) : 2,43 ppm : s : 3H ; 7,05-8,61 ppm : m : 7H ; 12,00 ppm : s : 1H.
Composé 134 : RMN DMSO (300 MHz) : 2,43 ppm : s : 3H ; 4,02 ppm : s : 3H ; 7,06 ppm : dd : 1H ; 7,29 ppm : se : 1H ; 7,35 ppm : d : 1H ; 7,72 ppm : s : 1H ; 7,84-7,94 ppm : m : 5H ; 8,45 ppm : s : 1H.
Composé 138 : RMN DMSO (300 MHz) : 1,71 ppm : s : 3H ; 2,44 ppm : s : 3H ; 2,64 ppm : s : 3H ; 4,02 ppm : s : 3H ; 4,20 ppm : s : 3H ; 7,12 ppm : d : 1H ; 7,48-7,56 ppm : m : 2H ; 7,76 ppm : m : 1H ; 7,87 ppm : d : 1H ; 8,03 ppm : d : 1H ; 8,35 ppm : m : 1H ; 8,46 ppm : s : 1H.
Composé 140 : RMN DMSO (300 MHz) : 2,79 ppm : s : 3H ; 3,69 ppm : s : 3H ; 6,83 ppm : t : 1H ; 7,03 ppm : dd : 1H ; 7,34 ppm : d : 1H ; 7,66-7,80 ppm : m : 5H ; 8,27 ppm : s : 1H ; 8,49 ppm : d : 1H ; 9,63 ppm : s : 1H ; 11,84 ppm : s : 1H.
Composé 141 : RMN DMSO (300 MHz) : 2,45 ppm : s : 3H ; 4,04 ppm : s : 3H ; 5,22 ppm : s : 2H ; 5,87 ppm : s : 2H ; 7,01-9,31 ppm : m : 8H.
Composé 142 : RMN DMSO (300 MHz) : 2,21 ppm : s : 6H ; 2,43 ppm : s : 3H ; 2,64 ppm : t : 2H ; 3,99 ppm : s : 3H ; 4,62 ppm : t : 2H ; 6,67-8,29 ppm : m : 8H ; 9,27 ppm : s : 1H.
Composé 145 : RMN DMSO/TFA (200 MHz) : 2,4 ppm : s : 3H ; 3,7 ppm : s : 3H ; 6,9-7,1 ppm : m : 2H ; 7,3-7,45 ppm : m : 3H ; 7,5-7,7 ppm : m : 2H ; 8,35 ppm : s : 1H.
Composé 148 : RMN DMSO (300 MHz) : 2,40 ppm : s : 3H ; 3,67 ppm : s : 3H ; 7,04 ppm : d : 1H ; 7,35 ppm : m : 2H ; 7,57 ppm : dd : 1H ; 7,63 ppm : s : 1H ; 7,77 ppm : d : 1H ; 8,12 ppm : s : 1H ; 11,96 ppm : s : 1H.
Composé 150 : RMN DMSO (300 MHz) : 2,43 ppm : m : 2H ; 2,51 ppm : s : 3H ; 4,10-4,30 ppm : m : 4H ; 7,17-8,20 ppm : m : 7H.
Composé 155 : RMN DMSO (300 MHz) : 1,36 ppm : t (J=7,0) : 3H ; 4,02 ppm : s : 3H ; 4,34 ppm : q (J=7,0) : 2H ; 5,97 ppm : s : 2H ; 7,48 ppm : m : 2H ; 7,70 ppm : s : 1H ; 7,88 ppm : d (J=8,6) : 1H ; 7,98 ppm : dd (J=1,5 ; J=8,6) : 1H ; 8,47 ppm : s : 1H ; 8,61 ppm : s : 1H.
Composé 156 : RMN DMSO (300 MHz) : 1,33 ppm : t (J=7) : 3H ; 3,70 ppm : s : 3H ; 4,32 ppm : q (J=7) : 2H ; 7,41 ppm : m : 2H ; 7,46 ppm : d (J=8,5) : 1H ; 7,67 ppm : s : 1H ; 7,86 ppm : d (J=8,5) : 1H ; 8,34 ppm : s : 1H ; 8,53 ppm : s : 1H ; 12,46 ppm : s : 1H.
Composé 159 : RMN DMSO (300 MHz) : 2,42 ppm : s : 3H ; 3,69 ppm : s : 3H ; 7,05 ppm : d (J=7,0) : 1H ; 7,21 ppm : d (J=7,29) : 1H ; 7,37 ppm : d (J=8,15) : 2H ; 7,51 ppm : d (J=2,6) : 1H ; 7,54 ppm : s : 1H ; 7,64 ppm : s : 1H ; 8,14 ppm : s : 1H ; 8,34 ppm : d (J=7,22) : 2H ; 10,71 ppm : s : 1H ; 12,03 ppm : s : 1H.
Composé 160 : RMN DMSO (300 MHz) : 2,40 ppm : s : 3H ; 3,17 ppm : m : 4H ; 3,66 ppm : s : 3H ; 3,75 ppm : m : 4H ; 6,94 ppm : dd (J=2,45 ; J=8,60) : 1H ; 7,03 ppm : m : 2H ; 7,23 ppm : d (J=8,51) : 1H ; 7,34 ppm : d (J=8,12) : 1H ; 7,62 ppm : s : 1H ; 8,02 ppm : s : 1H ; 11,87 ppm : s : 1H.
Composé 166 DMSO : 3,73 ppm : s : 3H ; 3,97 ppm : s : 3H ; 7,56 ppm : d : 1H ; 7,63 ppm : d : 1H ; 7,95 ppm : d : 1H ; 8,07 ppm : m : 2H ; 8,73 ppm : s : 1H ; 8,92 ppm : s : 1H ; 12,57 ppm : s : 1H.

Les composés selon l'invention ont fait l'objet d'essais pharmacologiques permettant de déterminer leur activité anticancéreuse.

Les composés de formule (I) selon la présente invention ont été testés *in vitro* sur une lignée cellulaire humaine de cancer de sein : la lignée MDA-MB-231 disponible auprès de l'American Type Culture Collection (référence HTB26).

L'évaluation de l'effet antiprolifératif est effectuée selon J.M. Derocq et al., FEBS Letters, 1998, 425, 419-425 : on mesure le taux d'incorporation de la [3H]thymidine dans l'ADN des cellules traitées, après 96 heures d'incubation d'un composé de formule (I). La concentration inhibitrice 50 (CI₅₀) est définie comme la concentration qui inhibe la prolifération cellulaire de 50 %.

Les composés selon l'invention présentent une CI₅₀ généralement inférieure à 10 µM sur la lignée MDA-MB-231.

Les composés de formule (I) ont également été testés sur une autre lignée cellulaire humaine de cancer du sein, dite lignée multi-résistante MDR, (de l'anglais multi-drug-resistant) et appelée MDA-A₁. Cette lignée est décrite par E. Collomb, C. Dussert et P.M. Martin dans Cytometry, 1991, 12(1), 15-25.

Le terme "multi-résistant" qui qualifie cette lignée, signifie que ladite lignée est peu sensible d'une manière générale aux drogues de chimiothérapie communément utilisée et en particulier aux antimitotiques d'origine naturelle tels que le paclitaxel, la vincristine, la vinblastine.

Les composés selon l'invention présentent une CI₅₀ généralement inférieure à 10 µM sur la lignée multi-résistante MDA-A₁.

Les composés selon l'invention ont également été testés *in vivo* dans des modèles murins de xénogreffes de tumeurs humaines selon les méthodes décrites dans la littérature : Mooberry SL et al., Int. J. Cancer, 2003, 104 (4), 512-521 ; Polin L et al., Invest. New Drugs, 2002, 20 (1), 13-22 ; Corbett TH et al., Invest. New Drugs, 1999, 17 (1), 17-27. Des fragments de tumeurs humaines de 2 à 3 mm de diamètre sont implantés de façon sous-cutanée chez des souris SCID (de l'anglais Severe Combined Immunodeficiency) de souche Balb/C (Iffa-Credo, Lyon, France). Lorsque ces tumeurs atteignent un poids de 50-60 mg, les composés sont administrés par voie orale ou intraveineuse tous les jours ou tous les deux jours pendant toute la durée de l'expérience (20 à 40 jours) à des doses variant de 10 à 300 mg/kg par administration. Le poids des tumeurs est estimé selon la formule : P (poids de la tumeur en mg) = (axb²)/2, où a et b représentent respectivement la longueur et la largeur en mm de l'implant tumoral. La mesure de a et de b est réalisée à l'aide d'un pied à coulisse. L'efficacité antitumorale est évaluée en comparant le poids moyen des tumeurs dans le groupe des animaux traités avec le composé à l'étude (T) à celui des animaux du groupe contrôle auxquels on a administré uniquement le solvant du composé (C). Cette mesure, exprimée en % du rapport T/C, est réalisée lorsque C atteint approximativement 1000 mg. Les composés selon l'invention ont démontré une activité antitumorale *in vivo* (rapport T/C inférieur à 100 %), certains de façon très significative avec un rapport T/C inférieur ou égal à 42 %.

Ainsi, selon la présente invention, il apparaît que les composés de formule (I) inhibent la prolifération des cellules tumorales y compris celle des cellules présentant une multi-résistance. Il apparaît donc que les composés selon l'invention ont une activité anticancéreuse.

Ainsi selon un autre de ses aspects, l'invention a pour objet des médicaments qui comprennent un composé de formule (I), ou un sel d'addition de ce dernier à un acide pharmaceutiquement acceptable ou encore un hydrate ou un solvat du composé de formule (I).

Ces médicaments trouvent leur emploi en thérapeutique, notamment dans le traitement ou la protection des maladies causées ou exacerbées par la prolifération des cellules tumorales.

Comme inhibiteur de la prolifération des cellules tumorales, ces composés sont utiles dans le traitement des tumeurs solides à la fois primaires et métastasiques, des carcinomes et cancers, en particulier : cancer du sein ; cancer du poumon ; cancer de l'intestin grêle, cancer du colon et du rectum ; cancer des voies respiratoires, de l'oropharynx et de l'hypopharynx ; cancer de l'oesophage; cancer du foie, cancer de l'estomac, cancer des canaux biliaires, cancer de la vésicule biliaire, cancer du pancréas; cancers des voies urinaires y compris rein, urothelium et vessie; cancers du tractus génital féminin y compris cancer de l'utérus, du col de l'utérus, des ovaires, chloriocarcinome et trophoblastome; cancers du tractus génital masculin y compris cancer de la prostate, des vésicules séminales, des testicules, tumeurs des cellules germinales; cancers des glandes endocrines y compris cancer de la thyroïde, de l'hypophyse, des glandes surrénales ; cancers de la peau y compris hémangiomes, mélanomes, sarcomes, incluant le sarcome de Kaposi ; tumeurs du cerveau, des nerfs, des yeux, des méninges, incluant astrocytomes, gliomes, glioblastomes, rétinoblastomes, neurinomes, neuroblastomes, schwannomes, méningiomes ; tumeurs solides venant de tumeurs malignes hématopoïétiques incluant leucémies, chloromes, plasmacytomes, mycosis fongoïde, lymphome ou leucémie des cellules T, lymphome non hodgkinien, hémopathies malignes, myélomes.

Selon un autre de ses aspects, la présente invention concerne des compositions pharmaceutiques comprenant, en tant que principe actif, un composé selon l'invention. Ces compositions pharmaceutiques contiennent une dose efficace d'au moins un composé selon l'invention, ou un sel pharmaceutiquement acceptable, un hydrate ou solvat dudit composé, ainsi qu'au moins un excipient pharmaceutiquement acceptable.

Lesdits excipients sont choisis selon la forme pharmaceutique et le mode d'administration souhaité, parmi les excipients habituels qui sont connus de l'Homme du métier.

Dans les compositions pharmaceutiques de la présente invention pour l'administration orale, sublinguale, sous-cutanée, intramusculaire, intra-veineuse, topique, locale, intratrachéale, intranasale, transdermique ou rectale, le principe actif de formule (I) ci-dessus, ou son sel, solvat ou hydrate éventuel, peut être administré sous forme unitaire d'administration, en mélange avec des excipients pharmaceutiques classiques, aux animaux et aux êtres humains pour la prophylaxie ou le traitement des troubles ou des maladies ci-dessus.

Les formes unitaires d'administration appropriées comprennent les formes par voie orale telles que les comprimés, les gélules molles ou dures, les poudres, les granules et les solutions ou suspensions orales, les formes d'administration sublinguale, buccale, intratrachéale, intraoculaire, intranasale, par inhalation, les formes d'administration topique, transdermique, sous-cutanée, intramusculaire ou intraveineuse, les formes d'administration rectale et les implants. Pour l'application topique, on peut utiliser les composés selon l'invention dans des crèmes, gels, pommades ou lotions.

Les composés de formule (I) ci-dessus peuvent être utilisés à des doses journalières de 0,002 à 2000 mg par kilogramme de poids corporel du mammifère à traiter, de préférence à des doses journalières de 0,1 à 300 mg/kg. Chez l'être humain, la dose peut varier de préférence de 0,02 à 10000 mg par jour, plus particulièrement de 1 à 3000 mg selon l'âge du sujet à traiter ou le type de traitement : prophylactique ou curatif.

Il peut y avoir des cas particuliers où des dosages plus élevés ou plus faibles sont appropriés ; de tels dosages ne sortent pas du cadre de l'invention. Selon la pratique habituelle, le dosage approprié à chaque patient est déterminé par le médecin selon le mode d'administration, le poids et la réponse dudit patient.

La présente invention, selon un autre de ses aspects, concerne également une méthode de traitement des pathologies ci-dessus indiquées qui comprend l'administration, à un patient, d'une dose efficace d'un composé selon l'invention, ou un de ses sels pharmaceutiquement acceptables ou hydrates ou solvats.

Selon la présente invention, le ou les composés de formule (I) peuvent être administrés en association avec un (ou plusieurs) principe(s) actif(s) anticancéreux, en particulier des composés antitumoraux tels que les agents alkylants tels que les alkylsulfonates (busulfan), la dacarbazine, la procarbazine, les moutardes azotées (chlorméthine, melphalan, chlorambucil), cyclophosphamide, ifosfamide; les nitrosourées tels que la carmustine, la lomustine, la sémustine, la streptozocine; les alcaloïdes antinéoplasiques tels que la vincristine, la vinblastine ; les taxanes tel que le paclitaxel ou le taxotère ; les antibiotiques antinéoplasiques tels que l'actinomycine; les agents intercalants, les antimétabolites antinéoplasiques, les antagonistes des folates, le méthotrexate; les inhibiteurs de la synthèse des purines; les analogues de la purine tels que mercaptopurine, 6-thioguanine; les inhibiteurs de la synthèse des pyrimidines, les inhibiteurs d'aromatase, la capécitabine, les analogues de la pyrimidine tels que fluorouracil, gemcitabine, cytarabine et cytosine arabinoside; le bréquinar ; les inhibiteurs de topoisomérases tels que la camptothécine ou l'étoposide ; les agonistes et antagonistes hormonaux anticancéreux incluant le tamoxifène; les inhibiteurs de kinase, l'imatinib; les inhibiteurs de facteurs de croissance; les antiinflammatoires tels que le pentosane polysulfate, les corticostéroïdes, la prednisone, la dexamethasone; les antitopoisomérases tels que l'étoposide, les antracyclines incluant la doxorubicine, la bléomycine, la mitomycine et la méthramycine; les complexes métalliques anticancéreux, les complexes du platine, le cisplatine, le carboplatine, l'oxaliplatine ; l'interféron alpha, le triphénylthiophosphoramide, l'altrétamine ; les agents antiangiogéniques ; la thalidomide ; les adjuvants d'immunothérapie ; les vaccins.

## Revendications

1. Composé répondant à la formule: 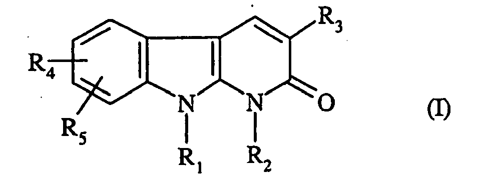 dans laquelle:
- R₁ représente un atome d'hydrogène, un groupe (C₁-C₄)alkyle ou un groupement (CH₂)ₙOH, (CH₂)ₙ-O-tétrahydropyran-2-yle, (CH₂)ₙNR'₆R'₇, (CH₂)ₙCN, (CH₂)ₙCO₂(C₁-C₄)Alk ou (CH₂)ₙCONR₆R₇ ;
- R₂ représente un atome d'hydrogène ou un groupe (C₁-C₄)alkyle ;
- ou R₁ et R₂ ensemble forment un groupe (CH₂)₃ ;
- R₃ représente un phényle monosubstitué par un groupe hydroxyle, hydroxyméthyle, carboxy, (C₁-C₄)alcanoyle, azido, (C₁-C₄)alcoxycarbonyle, hydroxyiminométhyle, (C₁-C₄)alkylsulfonyle, trifluorométhyle, thiole, (C₁-C₄)alkylthio, cyano ou par un groupement (CH₂)ₘNR'₇R₁₀, CONR₆R₈ ou O(CH₂)ₙR₉ ; un phényle substitué par 2 à 5 substituants identiques ou différents choisis parmi un atome d'halogène, un groupe (C₁-C₄)alkyle, trifluorométhyle, hydroxyle, hydroxyméthyle, (C₁-C₄)alcoxy, carboxy, (C₁-C₄)alcanoyle, azido, (C₁-C₄)alcoxycarbonyle, hydroxyiminométhyle, thiole, (C₁-C₄)alkylthio, (C₁-C₄)alkylsulfonyle, un phényle, cyano ou par un groupement (CH₂)ₘNR'₇R₁₀, CONR₆R₈ ou O(CH₂)ₙR₉ ; ou R₃ représente un groupe benzodioxolyle non substitué ou substitué sur le phényle par un atome d'halogène ;
- R₄ et R₅ sont identiques ou différents et représentent chacun indépendamment un atome d'hydrogène ou d'halogène ou un groupe hydroxyle, (C₁-C₄)alkyle, trifluorométhyle, phényle, cyano, (C₁-C₄)alcoxy, (C₁-C₄)alcoxycarbonyle, (C₁-C₄)alkylsulfonyle ou un groupement O-(CH₂)ₙNR₆R₇ ou (CH₂)ₙNR₆R₇ ;
- R₆ représente l'hydrogène ou un groupe (C₁-C₄)alkyle ;
- R₇ représente l'hydrogène ou un groupe (C₁-C₄)alkyle ;
- ou R₆ et R₇ ensemble avec l'atome d'azote auquel ils sont liés constituent un radical hétérocyclique choisi parmi : pipéridyle, morpholinyle, pyrrolidinyle, pipérazinyle ou 4-méthylpipérazin-1-yle ;
- R'₆ représente l'hydrogène ou un groupe (C₁-C₄)alkyle ;
- R'₇ représente l'hydrogène ou un groupe (C₁-C₄)alkyle ;
- ou R'₆ et R'₇ ensemble avec l'atome d'azote auquel ils sont liés constituent un radical hétérocyclique choisi parmi morpholinyle ou pyrrolidinyle ;
- R₈ représente l'hydrogène, un groupe (C₁-C₄)alkyle ou un groupement
- (CH₂)ₙNR₆R₇;
- ou R₆ et R₈ ensemble avec l'atome d'azote auquel ils sont liés constituent un radical hétérocyclique choisi parmi : pipéridyle, morpholinyle, pyrrolidinyle, pipérazinyle ou 4-méthylpipérazin-1-yle ;
- R₉ représente un radical phényle ou un groupe amino, morpholin-4-yle, cyano, ou (C₁-C₄)alcoxycarbonyle ;
- R₁₀ représente R'₆ ou un groupe phényle, pyridyle ou pyrimidinyle ou un groupement (CH₂)ₙNR'₆R'₇ ;
- ou R'₇ et R₁₀ ensemble avec l'atome d'azote auquel ils sont liés constituent un radical hétérocyclique choisi parmi pipérazinyle ou 4-méthylpipérazin-2-yle ;
- n représente 1, 2 ou 3 ;
- m représente 0 ou 1 ;
- Alk représente un alkyle :
à l'état de base ou de sel d'addition à un acide, ainsi qu'à l'état d'hydrate ou de solvat.

2. Composé de formule (I) selon la revendication 1, **caractérisé en ce que** :
- R₁ représente un atome d'hydrogène, un groupe méthyle, cyanométhyle, (C₁-C₄)alcoxycarbonylméthyle, aminométhyle, aminoéthyle, aminopropyle, pyrrolidinoéthyle ;
- et/ou R₂ représente un groupe méthyle;
- et/ou R₁ et R₂ ensemble forment un groupe (CH₂)₃ ;
et/ou R₃ représente un phényle monosubstitué par un groupe hydroxyle, (C₁-C₄)alcoxycarbonyle, méthylsulfonyle, trifluorométhyle, méthylthio, cyanométhoxy, aminoéthoxy, acétyle, hydroxyméthyle, cyano, amino, azido, aminométhyle, hydroxyiminométhyle ou un groupe (CH₂)ₘNR'₇R₁₀ dans lequel R'₇ représente un atome d'hydrogène ou un méthyle, R₁₀ représente un atome d'hydrogène ou un groupe phényle, pyridyle, pyrimidinyle ou R'₇ et R₁₀ ensemble avec l'atome d'azote auquel ils sont liés constituent un groupe pipérazin-1-yle ou 4-méthylpipérazin-1-yle et m représente zéro ou un ; ou R₃ représente un phényle substitué par 2 à 3 substituants identiques ou différents choisis parmi un atome d'halogène, un groupe méthyle, méthoxy, méthylthio, trifluorométhyle, hydroxyle, (C₁-C₄)alcoxycarbonyle, méthylsulfonyle, cyanométhoxy, aminoéthoxy, acétyle, hydroxyméthyle, cyano, amino, azido, aminométhyle, hydroxyiminométhyle ou un groupe (CH₂)ₘNR'₇R₁₀ dans lequel R'₇ représente un atome d'hydrogène ou un méthyle, R₁₀ représente un atome d'hydrogène ou un groupe phényle, pyridyle, pyrimidinyle ou R'₇ et R₁₀ ensemble avec l'atome d'azote auquel ils sont liés constituent un groupe pipérazin-1-yle ou 4-méthylpipérazin-1-yle et m représente zéro ou un ; ou R₃ représente un groupe benzodioxolyle non substitué ou substitué sur le phényle par un atome d'halogène;
- et/ou R₄ représente un atome d'halogène, un groupe méthyle, méthoxy ou (C₁-C₄)alcoxycarbonyle;
- et/ou R₅ représente un atome d'hydrogène ou un groupe méthyle;
à l'état de base ou de sel d'addition à un acide, ainsi qu'à l'état d'hydrate ou de solvat.

3. Composé de formule (I) selon la revendication 1 choisi parmi :
. Acide 3-(2,4-diméthoxyphényl)-1,9-diméthyl-2-oxo-2,9-dihydro-1*H*-pyrido[2,3-b]indole-6-carboxylique;
. 3-(2,4-diméthoxyphényl)-1,6-diméthyl-1,9-dihydro-2*H*-pyrido[2,3-b] indol-2-one;
. 3-(3-Hydroxyméthylphényl)-1,6-diméthyl-1,9-dihydro-2*H*-pyrido[2,3-b] indol-2-one.
3-(2,4-dichlorophényl)-1,6-diméthyl-1,9-dihydro-2*H*-pyrido[2,3-b] indol-2-one ;
. 3-(1,6-diméthyl-2-oxo-2,9-dihydro-1*H*-pyrido[2,3-b]indol-3-yl) benzonitrile ;
. 3-(4-Aminophényl)-1,6-diméthyl-1,9-dihydro-2*H*-pyrido[2,3-b]indol-2-one.
. 3-(6-chloro-1,3-benzodioxol-5-yl)-1,6-diméthyl-1,9-dihydro-2*H*-pyrido[2,3-b]indol-2-one ;
. 1,6-Diméthyl-1,9-dihydro-3-(phénylaminophényl)-2*H*-pyrido[2,3-b] indol-2-one.
. 6-bromo-3-(3,5-diméthylphényl)-1-méthyl-1,9-dihydro-2*H*-pyrido[2,3-b]indol-2-one ;
. 1,6-Diméthyl-3-(3-(trifluorométhyl)phényl)-1,9-dihydro-2*H*-pyrido[2,3-b] indol-2-one ;
. 1,6-Diméthyl-3-(3-(pyridin-2-ylamino)phényl)-1,9-dihydro-2*H*-pyrido[2,3-b] indol-2-one ;
. 1,6-Diméthyl-3-(3-pyrimidin-2-ylamino)phényl)-1,9-dihydro-2*H*-pyrido[2,3-b] indol-2-one ;
. 3-(3-Acétylphényl)-1,6-diméthyl-1,9-dihydro-2*H*-pyrido[2,3-b] indol-2-one ;
. 2-(2,4-Dichlorophényl)-9-méthyl-5,6-dihydro-3*H*, 4*H*-3a, 6a-diazafluoroanthen-3-one ;
9-(Cyanométhyl)-3-(2,4-dichlorophényl)-2-oxo-2,9-dihydro-1*H*-pyrido[2,3-b]indole-6-carboxylate de méthyle.
à l'état de base ou de sel d'addition à un acide, ainsi qu'à l'état d'hydrate ou de solvat.

4. Procédé de préparation d'un composé de formule (I) selon l'une des revendications 1 à 3, **caractérisé en ce que** :
on fait réagir un 2-aminoindole de formule : 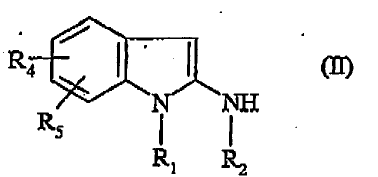
dans laquelle R₁, R₂, R₄ et R₅ sont tels que définis pour un composé de formule
(I) avec un ester de formule : 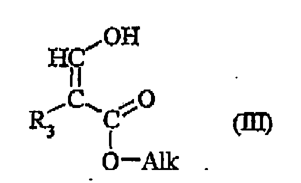
dans laquelle R₃ est tel que défini pour un composé de formule (I) et Alk représente un alkyle en C₁-C₄.

5. Procédé de préparation d'un composé de formule (I) selon l'une des revendications 1 à 3, **caractérisé en ce que** :
on fait réagir un aminoindole de formule : 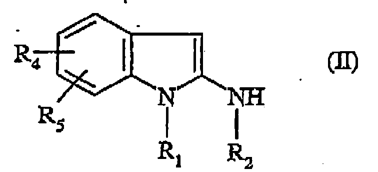
dans laquelle R₁, R₂, R₄ et R₅ sont tels que définis pour un composé de formule (I) avec un ester de formule; 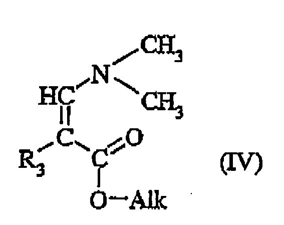 dans laquelle R₃ est tel que défini pour un composé de formule (I) et Alk représente un alkyle en C₁-C₄.

6. Médicament, **caractérisé en ce qu'**il comprend un composé de formule (I) selon l'une quelconque des revendications 1 à 3, ou un sel d'addition de ce composé à un acide pharmaceutiquement acceptable, ou encore un hydrate ou un solvat du composé de formule (I).

7. Composition pharmaceutique, **caractérisée en ce qu'**elle comprend un composé de formule (I) selon l'une quelconque des revendications 1 à 3, ou un sel pharmaceutiquement acceptable, un hydrate ou un solvat de ce composé, ainsi qu'au moins un excipient pharmaceutiquement acceptable.

8. Utilisation d'un composé de formule (I) selon l'une quelconque des revendications 1 à 3, pour la préparation d'un médicament destiné au traitement et à le prévention de maladies causées ou exacerbées par la prolifération des cellules tumorales.

## Claims

1. Compound corresponding to the formula: 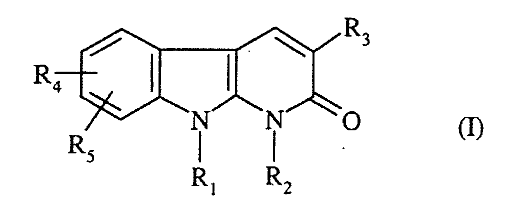 in which:
- R₁ represents a hydrogen atom, a (C₁-C₄)alkyl group or a (CH₂)ₙOH, (CH₂)ₙ-O-tetrahydropyran-2-yl, (CH₂)ₙNR'₆R'₇, (CH₂)ₙCN, (CH₂)ₙCO₂(C₁-C₄)alk or (CH₂)ₙCONR₆R₇ group;
- R₂ represents a hydrogen atom or a (C₁-C₄)alkyl group;
- or R₁ and R₂ together form a (CH₂)₃ group;
- R₃ represents a phenyl monosubstituted by a hydroxyl, hydroxymethyl, carboxyl, (C₁-C₄)alkanoyl, azido, (C₁-C₄)alkoxycarbonyl, hydroxyiminomethyl, (C₁-C₄)alkylsulphonyl, trifluoromethyl, thiol, (C₁-C₄)alkylthio or cyano group or by a (CH₂)ₘNR'₇R₁₀, CONR₆R₈ or O(CH₂)ₙR₉ group; a phenyl substituted by 2 to 5 identical or different substituents chosen from a halogen atom, a (C₁-C₄)alkyl, trifluoromethyl, hydroxyl, hydroxymethyl, (C₁-C₄)alkoxy, carboxyl, (C₁-C₄)alkanoyl, azido, (C₁-C₄)alkoxycarbonyl, hydroxyiminomethyl, thiol, (C₁-C₄)alkylthio or (C₁-C₄)alkylsulphonyl group, or a phenyl or cyano, or by a (CH₂)ₘNR'₇R₁₀, CONR₆R₈ or O(CH₂)ₙR₉ group; or R₃ represents a benzodioxolyl group which is unsubstituted or substituted on the phenyl by a halogen atom;
- R₄ and R₅ are identical or different and each independently represent a hydrogen or halogen atom or a hydroxyl, (C₁-C₄)alkyl, trifluoromethyl, phenyl, cyano, (C₁-C₄)alkoxy, (C₁-C₄)alkoxycarbonyl or (C₁-C₄)alkylsulphonyl group or an O-(CH₂)ₙNR₆R₇ or (CH₂)ₙNR₆R₇ group;
- R₆ represents hydrogen or a (C₁-C₄)alkyl group;
- R₇ represents hydrogen or a (C₁-C₄)alkyl group;
- or R₆ and R₇, together with the nitrogen atom to which they are bonded, form a heterocyclic radical chosen from: piperidyl, morpholinyl, pyrrolidinyl, piperazinyl or 4-methylpiperazin-1-yl;
- R'₆ represents hydrogen or a (C₁-C₄)alkyl group;
- R'₇ represents hydrogen or a (C₁-C₄)alkyl group;
- or R'₆ and R'₇, together with the nitrogen atom to which they are bonded, form a heterocyclic radical chosen from morpholinyl or pyrrolidinyl;
- R₈ represents hydrogen, a (C₁-C₄) alkyl group or a
- (CH₂)ₙNR₆R₇ group;
- or R₆ and R₈, together with the nitrogen atom to which they are bonded, form a heterocyclic radical chosen from: piperidyl, morpholinyl, pyrrolidinyl, piperazinyl or 4-methylpiperazin-1-yl;
- R₉ represents a phenyl radical or an amino, morpholin-4-yl, cyano or (C₁-C₄)alkoxycarbonyl group;
- R₁₀ represents R'₆ or a phenyl, pyridyl or pyrimidinyl group or a (CH₂)ₙNR'₆R'₇ group;
- or R'₇ and R₁₀, together with the nitrogen atom to which they are bonded, form a heterocyclic radical chosen from piperazinyl or 4-methylpiperazin-2-yl;
- n represents 1, 2 or 3;
- m represents 0 or 1;
- Alk represents an alkyl;
in the base form or in the form of an addition salt with an acid, and in the form of a hydrate or of a solvate.

2. Compound of formula (I) according to Claim 1, **characterized in that**:
- R₁ represents a hydrogen atom or a methyl, cyanomethyl, (C₁-C₄)alkoxycarbonylmethyl, aminomethyl, aminoethyl, aminopropyl or pyrrolidinoethyl group;
- and/or R₂ represents a methyl group;
- and/or R₁ and R₂ together form a (CH₂)₃ group;
- and/or R₃ represents a phenyl monosubstituted by a hydroxyl, (C₁-C₄)alkoxycarbonyl, methylsulphonyl, trifluoromethyl, methylthio, cyanomethoxy, aminoethoxy, acetyl, hydroxymethyl, cyano, amino, azido, aminomethyl or hydroxyiminomethyl group or a (CH₂)ₘNR'₇R₁₀ group in which R'₇ represents a hydrogen atom or a methyl, R₁₀ represents a hydrogen atom or a phenyl, pyridyl or pyrimidinyl group or R'₇ and R₁₀, together with the nitrogen atom to which they are bonded, form a piperazin-1-yl or 4-methylpiperazin-1-yl group, and m represents zero or one; or R₃ represents a phenyl substituted by 2 to 3 identical or different substituents chosen from a halogen atom, a methyl, methoxy, methylthio, trifluoromethyl, hydroxyl, (C₁-C₉)alkoxycarbonyl, methylsulphonyl, cyanomethoxy, aminoethoxy, acetyl, hydroxymethyl, cyano, amino, azido, aminomethyl or hydroxyiminomethyl group or a (CH₂)ₘNR'₇R₁₀ group in which R'₇ represents a hydrogen atom or a methyl, R₁₀ represents a hydrogen atom or a phenyl, pyridyl or pyrimidinyl group or R'₇ and R₁₀, together with the nitrogen atom to which they are bonded, form a piperazin-1-yl or 4-methylpiperazin-1-yl group, and m represents zero or one; or R₃ represents a benzodioxolyl group which is unsubstituted or substituted on the phenyl by a halogen atom;
- and/or R₄ represents a halogen atom or a methyl, methoxy or (C₁-C₄)alkoxycarbonyl group;
- and/or R₅ represents a hydrogen atom or a methyl group;
in the base form or in the form of an addition salt with an acid, and in the form of a hydrate or of a solvate.

3. Compound of formula (I) according to Claim 1, chosen from:
• 3-(2,4-dimethoxyphenyl)-1,9-dimethyl-2-oxo-2,9-dihydro-1*H*-pyrido[2,3-b]indole-6-carboxylic acid;
• 3-(2,4-dimethoxyphenyl)-1,6-dimethyl-1,9-dihydro-2*H*-pyrido[2,3-b]indol-2-one;
• 3-(3-hydroxymethylphenyl)-1,6-dimethyl-1,9-dihydro-2*H*-pyrido[2,3-b]indol-2-one;
• 3-(2,4-dichlorophenyl)-1,6-dimethyl-1,9-dihydro-2*H*-pyrido[2,3-b]indol-2-one;
• 3-(1,6-dimethyl-2-oxo-2,9-dihydro-1*H-*pyrido[2,3-b]-indol-3-yl)benzonitrile;
• 3-(4-aminophenyl)-1,6-dimethyl-1,9-dihydro-2*H-*pyrido[2,3-b]indol-2-one;
• 3-(6-chloro-1,3-benzodioxol-5-yl)-1,6-dimethyl-1,9-dihydro-2*H*-pyrido[2,3-b]indol-2-one;
• 1,6-dimethyl-1,9-dihydro-3-(phenylaminophenyl)-2*H*-pyrido[2,3-b]indol-2-one;
• 6-bromo-3-(3,5-dimethylphenyl)-1-methyl-1,9-dihydro-2*H*-pyrido[2,3-b]indol-2-one;
• 1,6-dimethyl-3-(3-(trifluoromethyl)phenyl)-1,9-dihydro-2*H*-pyrido[2,3-b]indol-2-one;
• 1,6-dimethyl-3-(3-(pyridin-2-ylamino)phenyl)-1,9-dihydro-2*H*-pyrido[2,3-b]indol-2-one;
• 1,6-dimethyl-3-(3-(pyrimidin-2-ylamino)phenyl)-1,9-dihydro-2*H*-pyrido[2,3-b]indol-2-one;
• 3-(3-acetylphenyl)-1,6-dimethyl-1,9-dihydro-2*H-*pyrido[2,3-b]indol-2-one;
• 2-(2,4-dichlorophenyl)-9-methyl-5,6-dihydro-3*H*,4*H*-3a,6a-diazafluoranthen-3-one;
• methyl 9-(cyanomethyl)-3-(2,4-dichlorophenyl)-2-oxo-2,9-dihydro-1*H*-pyrido[2,3-b]indole-6-carboxylate;
in the base form or in the form of an addition salt with an acid, and in the form of a hydrate or of a solvate.

4. Process for the preparation of a compound of formula (I) according to one of Claims 1 to 3, **characterized in that**:
a 2-aminoindole of formula: 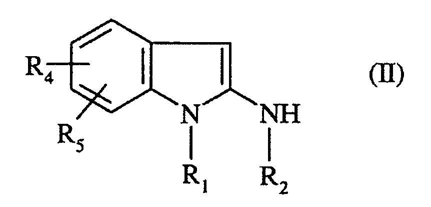
in which R₁, R₂, R₄ and R₅ are as defined for a compound of formula (I), is reacted with an ester of formula: 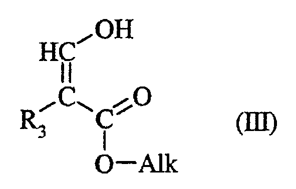 in which R₃ is as defined for a compound of formula (I) and Alk represents a C₁-C₄ alkyl.

5. Process for the preparation of a compound of formula (I) according to one of Claims 1 to 3, **characterized in that**:
an aminoindole of formula: 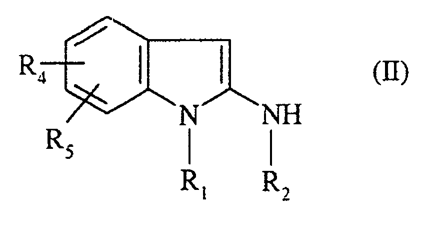
in which R₁, R₂, R₄ and R₅ are as defined for a compound of formula (I), is reacted with an ester of formula: 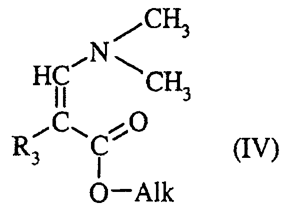 in which R₃ is as defined for a compound of formula (I) and Alk represents a C₁-C₄ alkyl.

6. Medicament, **characterized in that** it comprises a compound of formula (I) according to any one of Claims 1 to 3 or an addition salt of this compound with a pharmaceutically acceptable acid or also a hydrate or a solvate of the compound of formula (I).

7. Pharmaceutical composition, **characterized in that** it comprises a compound of formula (I) according to any one of Claims 1 to 3, or a pharmaceutically acceptable salt, a hydrate or a solvate of this compound, and at least one pharmaceutically acceptable excipient.

8. Use of a compound of formula (I) according to any one of Claims 1 to 3 for the preparation of a medicament intended for the treatment and prevention of diseases caused or exacerbated by the proliferation of tumour cells.

## Patentansprüche

1. Verbindung der Formel: 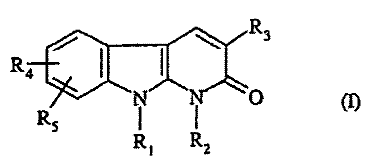 worin:
- R₁ ein Wasserstoffatom, eine (C₁-C₄)-Alkylgruppe oder eine Gruppe (CH₂)ₙOH, (CH₂)ₙ-O-Tetrahydropyran-2-yl, (CH₂)ₙNR'₆R'₇, (CH₂)ₙCN, (CH₂)ₙCO₂(C₁-C₄)Alk oder (CH₂)ₙCONR₆R₇ darstellt;
- R₂ ein Wasserstoffatom oder eine (C₁-C₄)-Alkylgruppe darstellt;
- oder R₁ und R₂ zusammen eine (CH₂)₃-Gruppe bilden;
- R₃ ein Phenyl darstellt, das mit einer Hydroxyl-, Hydroxymethyl-, Carboxy-, (C₁-C₄)-Alkanoyl-, Azido-, (C₁-C₄)-Alkoxycarbonyl-, Hydroxyiminomethyl-, (C₁-C₄)-Alkylsulfonyl-, Trifluormethyl-, Thiol-, (C₁-C₄)-Alkylthio-, Cyanogruppe oder mit einer Gruppe (CH₂)ₘNR'₇R₁₀, CONR₆R₈ oder O(CH₂)ₙR₉ monosubstituiert ist; ein Phenyl, das mit 2 bis 5 gleichen oder verschiedenen Substituenten substituiert ist, die aus einem Halogenatom, einer (C₁-C₄)-Alkyl-, Trifluormethyl-, Hydroxyl-, Hydroxymethyl-, (C₁-C₄) -Alkoxy-, Carboxy-, (C₁-C₄) -Alkanoyl-, Azido-, (C₁-C₄)-Alkoxycarbonyl-, Hydroxyiminomethyl-, Thiol-, (C₁-C₄)-Alkylthio-, (C₁-C₄)-Alkylsulfonyl-, Phenyl-, Cyanogruppe oder einer Gruppe (CH₂)ₘNR'₇R₁₀, CONR₆R₈ oder O(CH₂)ₙR₉ ausgewählt sind; oder R₃ eine Benzodioxolylgruppe darstellt, die unsubstituiert oder an dem Phenyl mit einem Halogenatom substituiert ist;
- R₄ und R₅ gleich oder verschieden sind und jeweils unabhängig ein Wasserstoff- oder Halogenatom oder eine Hydroxyl-, (C₁-C₄)-Alkyl-, Trifluormethyl-, Phenyl-, Cyano-, (C₁-C₄)-Alkoxy-, (C₁-C₉)-Alkoxycarbonyl-, (C₁-C₄)-Alkylsulfonylgruppe oder eine Gruppe O(CH₂)ₙNR₆R₇ oder (CH₂)ₙNR₆R₇ darstellen;
- R₆ Wasserstoff oder eine (C₁-C₄) -Alkylgruppe darstellt;
- R₇ Wasserstoff oder eine (C₁-C₄)-Alkylgruppe darstellt;
- oder R₆ und R₇, zusammen mit dem Stickstoffatom, aus das sie gebunden sind, einen heterocyclischen Rest bilden, ausgewählt aus: Piperidyl, Morpholinyl, Pyrrolidinyl, Piperazinyl oder 4-Methylpiperazin-1-yl;
- R'₆ Wasserstoff oder eine (C₁-C₄)-Alkylgruppe darstellt;
- R'₇ Wasserstoff oder eine (C₁-C₉)-Alkylgruppe darstellt;
- oder R'₆ und R'₇ zusammen mit dem Stickstoffatom, an das sie gebunden sind, einen heterocyclischen Rest bilden, ausgewählt aus Morpholinyl oder Pyrrolidinyl;
- R₈ Wasserstoff, eine (C₁-C₄)-Alkylgruppe oder eine Gruppe (CH₂)ₙNR₆R₇ darstellt;
- oder R₆ und R₈ zusammen mit dem Stickstoffatom, an das sie gebunden sind, einen heterocyclischen Rest bilden, ausgewählt aus: Piperidyl, Morpholinyl, Pyrrolidinyl, Piperazinyl oder 4-Methylpiperazin-1-yl;
- R₉ einen Phenylrest oder eine Amino-, Morpholin-4-yl-, Cyano- oder (C₁-C₄)-Alkoxycarbonylgruppe darstellt;
- R₁₀ für R'₆ oder eine Phenyl-, Pyridyl- oder Pyrimidinylgruppe oder eine Gruppe (CH₂)ₙNR'₆R'₇ steht;
- oder R'₇ und R₁₀ zusammen mit dem Stickstoffatom, an das sie gebunden sind, einen heterocyclischen Rest bilden, ausgewählt aus Piperazinyl oder 4-Methylpiperazin-2-yl;
- n 1, 2 oder 3 darstellt;
- m 0 oder 1 darstellt;
- Alk ein Alkyl darstellt;
im Zustand der Base oder eines Säureadditionssalzes sowie im Zustand eines Hydrats oder Solvats.

2. Verbindung der Formel (I) nach Anspruch 1, **dadurch gekennzeichnet, dass**:
- R₁ ein Wasserstoffatom oder eine Methyl-, Cyanomethyl-, (C₁-C₄)-Alkoxycarbonylmethyl-, Aminomethyl-, Aminoethyl-, Aminopropyl- oder Pyrrolidinoethylgruppe darstellt;
- und/oder R₂ eine Methylgruppe darstellt;
- und/oder R₁ und R₂ zusammen eine (CH₂)₃-Gruppe bilden;
- und/oder R₃ ein Phenyl darstellt, das mit einer Hydroxyl-, (C₁-C₄)-Alkoxycarbonyl-, Methylsulfonyl-, Trifluormethyl-, Methylthio-, Cyanomethoxy-, Aminoethoxy-, Acetyl-, Hydroxymethyl-, Cyano-, Amino-, Azido-, Aminomethyl- oder Hydroxyiminomethylgruppe oder einer Gruppe (CH₂)ₘNR'₇R₁₀ monosubstituiert ist, worin R'₇ ein Wasserstoffatom oder ein Methyl darstellt, R₁₀ ein Wasserstoffatom oder eine Phenyl-, Pyridyl-, Pyrimidinylgruppe darstellt, oder R'₇ und R₁₀ zusammen mit dem Stickstoffatom, an das sie gebunden sind, eine Piperazin-1-yl oder 4-Methylpiperazin-1-yl-Gruppe bilden und m null oder eins darstellt; oder R₃ ein Phenyl darstellt, das mit 2 bis 3 gleichen oder verschiedenen Substituenten substituiert ist, ausgewählt aus einem Halogenatom, einer Methyl-, Methoxy-, Methylthio-, Trifluormethyl-, Hydroxyl-, (C₁-C₄)-Alkoxycarbonyl-, Methylsulfonyl-, Cyanomethoxy-, Aminoethoxy-, Acetyl-, Hydroxymethyl-, Cyano-, Amino-, Azido-, Aminomethyl-, Hydroxyiminomethylgruppe oder einer Gruppe (CH₂)ₘNR'₇R₁₀, worin R'₇ ein Wasserstoffatom oder ein Methyl darstellt, R₁₀ ein Wasserstoffatom oder eine Phenyl-, Pyridyl-, Pyrimidinylgruppe darstellt oder R'₇ und R₁₀ zusammen mit dem Stickstoffatom, an das sie gebunden sind, eine Piperazin-1-yl- oder 4-Methylpiperazin-1-yl-Gruppe bilden und m null oder eins darstellt; oder R₃ eine Benzodioxolylgruppe darstellt, die unsubstituiert oder an dem Phenyl mit einem Halogenatom substituiert ist;
- und/oder R₄ ein Halogenatom, eine Methyl-, Methoxy- oder (C₁-C₉)-Alkoxycarbonylgruppe darstellt;
- und/oder R₅ ein Wasserstoffatom oder eine Methylgruppe darstellt;
im Zustand der Base oder eines Säureadditionssalzes sowie im Zustand eines Hydrats oder Solvats.

3. Verbindung der Formel (I) nach Anspruch 1, ausgewählt aus:
3-(2,4-Dimethoxyphenyl)-1,9-dimethyl-2-oxo-2,9-dihydro-1H-pyrido-[2,3-b]-indol-6-carbonsäure;
3-(2,4-Dimethoxyphenyl)-1,6-dimethyl-1,9-dihydro-2*H*-pyrido-[2,3-b]-indol-2-on;
3-(3-Hydroxymethylphenyl)-1,6-dimethyl-1,9-dihydro-2*H*-pyrido-[2,3-b]-indol-2-on;
3-(2,4-Dichlorphenyl)-1,6-dimethyl-1,9-dihydro-2*H-*pyrido-[2,3-b]-indol-2-on;
3-(1,6-Dimethyl-2-oxo-2,9-dihydro-1H-pyrido-[2,3-b]-indol-3-yl)-benzonitril;
3-(4-Aminophenyl)-1,6-dimethyl-1,9-dihydro-2*H-*pyrido-[2,3-b]-indol-2-on;
3-(6-Chlor-1,3-benzodioxol-5-yl)-1,6-dimethyl-1,9-dihydro-2*H*-pyrido-[2,3-b]-indol-2-on;
1,6-Dimethyl-1,9-dihydro-3-(phenylaminophenyl)-2*H-*pyrido-[2,3-b]-indol-2-on;
6-Brom-3-(3,5-dimethylphenyl)-1-methyl-1,9-dihydro-2*H*-pyrido-[2,3-b]-indol-2-on;
1,6-Dimethyl-3-(3-(trifluormethyl)phenyl)-1,9-dihydro-2*H*-pyrido-[2,3-b]-indol-2-on;
1,6-Dimethyl-3-(3-(pyridin-2-ylamino)phenyl)-1,9-dihydro-2*H*-pyrido-[2,3-b]-indol-2-on;
1,6-Dimethyl-3-(3-(pyrimidin-2-ylamino)phenyl)-1,9-dihydro-2H-pyrido-[2,3-b]-indol-2-on;
3-(3-Acetylphenyl)-1,6-dimethyl-1,9-dihydro-2*H-*pyrido-[2,3-b]-indol-2-on;
2-(2,4-Dichlorphenyl)-9-methyl-5,6-dihydro-3*H*,4*H-*3a,6a-diazafluoranthen-3-on oder
Methyl-9-(cyanomethyl)-3-(2,4-dichlorphenyl)-2-oxo-2,9-dihydro-1*H*-pyrido-[2,3-b]-indol-6-carboxylat im Zustand der Base oder eines Säureadditionssalzes sowie im Zustand eines Hydrats oder Solvats.

4. Verfahren zur Herstellung einer Verbindung der Formel (I) nach einem der Ansprüche 1 bis 3, **dadurch gekennzeichnet, dass**:
man ein 2-Aminoindol der Formel: 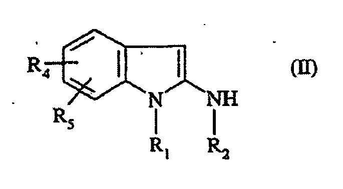
worin R₁, R₂, R₄ und R₅ wie für eine Verbindung der Formel (I) definiert sind, mit einem Ester der Formel: 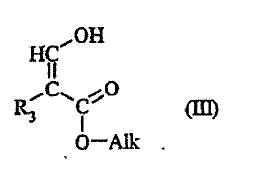 umsetzt, worin R₃ wie für eine Verbindung der Formel (I) definiert ist und Alk ein C₁-C₄-Alkyl darstellt.

5. Verfahren zur Herstellung einer Verbindung der Formel (I) nach einem der Ansprüche 1 bis 3, **dadurch gekennzeichnet, dass**:
man ein Aminoindol der Formel: 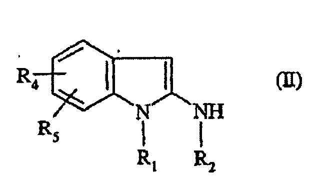
worin R₁, R₂, R₄ und R₅ wie für eine Verbindung der Formel (I) definiert sind, mit einem Ester der Formel: 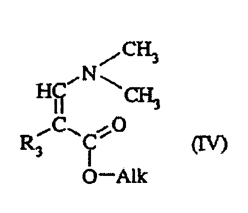 umsetzt, worin R₃ wie für eine Verbindung der Formel (I) definiert ist und Alk ein C₁-C₄-Alkyl darstellt.

6. Arzneimittel, **dadurch gekennzeichnet, dass** es eine Verbindung der Formel (I) nach einem der Ansprüche 1 bis 3 oder ein Additionssalz dieser Verbindung mit einer pharmazeutisch annehmbaren Säure oder auch ein Hydrat oder ein Solvat der Verbindung der Formel (I) umfasst.

7. Pharmazeutische Zusammensetzung, **dadurch gekennzeichnet, dass** sie eine Verbindung der Formel (I) nach einem der Ansprüche 1 bis 3 oder ein pharmazeutisch annehmbares Salz, ein Hydrat oder ein Solvat dieser Verbindung sowie mindestens einen pharmazeutisch annehmbaren Exzipienten umfasst.

8. Verwendung einer Verbindung der Formel (I) nach einem der Ansprüche 1 bis 3 zur Herstellung eines Arzneimittels, das zur Behandlung und Vorbeugung von Erkrankungen bestimmt ist, die durch Proliferation von Tumorzellen verursacht oder verschlimmert werden.
